(19) **Europäisches Patentamt**
**European Patent Office**
**Office européen des brevets**

(11) **EP 1 720 506 B1**

(12) **EUROPÄISCHE PATENTSCHRIFT**

(45) Veröffentlichungstag und Bekanntmachung des Hinweises auf die Patenterteilung:
**26.11.2008 Patentblatt 2008/48**

(51) Int Cl.:
**A61K 6/083** (2006.01)

(21) Anmeldenummer: **05715666.3**

(22) Anmeldetag: **02.03.2005**

(86) Internationale Anmeldenummer:
**PCT/EP2005/002196**

(87) Internationale Veröffentlichungsnummer:
**WO 2005/084611 (15.09.2005 Gazette 2005/37)**

(54) **GEFÜLLTES, POLYMERISIERBARES DENTALMATERIAL UND VERFAHREN ZU DESSEN HERSTELLUNG**

FILLED POLYMERISABLE DENTAL MATERIAL AND METHOD FOR THE PRODUCTION THEREOF

MATERIAU DENTAIRE CHARGE POLYMERISABLE ET PROCEDE POUR LE PRODUIRE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **02.03.2004 EP 04004852**

(43) Veröffentlichungstag der Anmeldung:
**15.11.2006 Patentblatt 2006/46**

(73) Patentinhaber: **Ernst Mühlbauer GmbH & Co.KG 25870 Norderfriedrichskoog (DE)**

(72) Erfinder:
• **NEFFGEN, Stephan**
  **22459 Hamburg (DE)**
• **HAUSER, Karsten**
  **22529 Hamburg (DE)**
• **SEBALD, Monika**
  **27721 Ritterhude (DE)**
• **HARTWIG, Andreas**
  **27721 Ritterhude (DE)**

(74) Vertreter: **Glawe, Delfs, Moll Patent- und Rechtsanwälte Rothenbaumchaussee 58 20148 Hamburg (DE)**

(56) Entgegenhaltungen:
**EP-A- 0 530 926       EP-A- 0 648 484**
**EP-A- 1 149 573       WO-A-02/092022**
**DE-A- 10 042 050       DE-A- 19 617 931**
**US-A- 6 121 344**

• **PATENT ABSTRACTS OF JAPAN Bd. 1997, Nr. 11, 28. November 1997 (1997-11-28) & JP 09 194674 A (SAN MEDICAL KK), 29. Juli 1997 (1997-07-29)**
• **PATENT ABSTRACTS OF JAPAN Bd. 1996, Nr. 10, 31. Oktober 1996 (1996-10-31) & JP 08 143747 A (SAN MEDICAL KK), 4. Juni 1996 (1996-06-04)**

**Beschreibung**

Die Erfindung betrifft ein gefülltes und polymerisierbares Dentalmaterial sowie ein Verfahren zu dessen Herstellung.

Für die prothetische, konservierende und präventive Zahnheilkunde werden verschiedene Dentalmaterialien verwendet. Dazu gehören auch sogenannte Komposite, die ein oder mehrere Füllmaterialien in einer Harzmatrix aufweisen. Die Füllstoffe verleihen den Dentalmaterialien die gewünschten mechanischen Eigenschaften, dazu gehören die Rheologie in der Verarbeitungsphase und mechanische Eigenschaften wie Härte oder Abriebfestigkeit im ausgehärteten Zustand. Es ist bereits vorgeschlagen worden (WO-A-020 92 022), die mechanischen Eigenschaften von Dentalmaterialien durch Zusatz von nanoskaligen Füllstoffen zu verbessern. Die in der genannten Schrift offenbarten Nanofüllstoffe werden durch ein sehr aufwendiges Plasmafackelverfahren (PVS) hergestellt.

Der vorliegenden Erfindung liegt die Aufgabe zu Grunde, gefüllte und polymerisierbare Dentalmaterialien sowie ein Verfahren zu deren Herstellung zu schaffen, wobei die Dentalmaterialien kostengünstig hergestellt werden können und gute mechanische Eigenschaften wie beispielsweise Druckfestigkeit und Abriebfestigkeit aufweisen.

Die Erfindung löst diese Aufgabe durch ein gefülltes und polymerisierbares Dentalmaterial, das enthält:

a) ein organisches Bindemittel,

b) einen nanoskaligen Füllstoff, der folgende Merkmale aufweist:

-   mindestens 50 Gew.-%, bevorzugt mindestens 60 Gew.-% und besonders bevorzugt mindestens 80 Gew.-% der Nanopartikel weisen einen Teilchendurchmesser von kleiner 200 nm, vorzugsweise weniger als 150 bzw. 100 nm, auf,
-   mindestens 20 Partikelzahl-%, bevorzugt mindestens 30 Partikelzahl-%, bevorzugt mindestens 40 Partikelzahl-% und besonders bevorzugt mindestens 50 Partikelzahl-% der Nanopartikel sind aggregierte Teilchen,
-   der nanoskalige Füllstoff ist organisch oberflächenmodifiziert

c) wenigstens einen anorganischen und/oder organischen Füllstoff ausgewählt aus der Gruppe bestehend aus einem gemahlenen Füllstoff mit einer mittleren Korngröße zwischen 0,2 $\mu$m und 50 $\mu$m und einem sphärischen Füllstoff mit einer mittleren Korngröße zwischen 0,1 $\mu$m und 50 $\mu$m.

Das erfindungsgemäße Verfahren zur Herstellung eines solchen Dentalmaterials weist folgende Schritte auf:

a) zur Verfügung stellen von:

a1) einem organischem Bindemittel,

a2) einem wenigstens teilweise agglomerierten und/oder aggregierten nanoskaligen Füllstoff,

a3) einem Mittel zur organischen Oberflächenmodifikation des organischen Füllstoffs a2),

a4) wenigstens einem anorganischen und/oder organischen Füllstoff ausgewählt aus der Gruppe bestehend aus einem gemahlenen Füllstoff mit einer mittleren Korngröße zwischen 0,2 $\mu$m und 50 $\mu$m und einem sphärischen Füllstoff mit einer mittleren Korngröße zwischen 0,1 $\mu$m und 50 $\mu$m;

b) Durchführung einer organischen Oberflächenmodifikation des Füllstoffs a2) mit dem Mittel a3);

c) Einarbeiten des oberflächenmodifizierten nanoskaligen Füllstoffs in das organische Bindemittel bis wenigstens 50 %, vorzugsweise wenigstens 60 %, weiter vorzugsweise wenigstens 80 % des nanoskaligen Füllstoffs einen Teilchendurchmesser von weniger als 200 nm, vorzugsweise weniger als 150 bzw. 100 nm, aufweisen;

d) Einarbeiten des Füllstoffs a4) in das organische Bindemittel;

wobei die Schritte c) und d) in beliebiger Reihenfolge oder gleichzeitig durchgeführt werden können und wobei Schritt b) vor oder gleichzeitig mit den Schritten c) und/oder d) durchgeführt wird.

Kern der Erfindung ist Kombination eines in Merkmal b) des Anspruchs 1 näher definierten kostengünstig gewinnbaren nanoskaligen Füllstoffs mit einem weiteren, in Merkmal c) des Anspruchs 1 näher definierten (Mikro) Füllstoff. Nanopartikel können durch den Sol-Gel-Prozess hergestellt werden. Bei diesem Verfahren werden Alkoxysilane hydrolisiert und die gebildeten Silanole kondensieren verhältnismäßig langsam zu Nanopartikeln. Es entstehen im Wesentlichen lediglich sogenannte Primärpartikel mit sehr einheitlicher Größenverteilung. Das Verfahren ist sehr teuer, da die Partikel vollständig aus Silanen hergestellt werden müssen.

Die flammpyrolytische Herstellung von Kieselsäure ist ebenfalls bekannt. Es können dabei ebenfalls Primärpartikel im Nanobereich entstehen. Unter den Reaktionsbedingungen der Flammenpyrolyse entstehen aus den Primärpartikeln jedoch zu einem großen Teil sogenannte Agglomerate oder Aggregate. Der Begriff Aggregate bezeichnet Teilchen, bei denen zwei oder mehr Primärteilchen über starke Bindungen wie bspw. Sinterbrücken miteinander assoziiert sind. Agglomerate sind Teilchen, bei denen zwei oder mehr Primärteilchen und/oder Aggregate über verhältnismäßig schwache Bindungen wie z. B. Wasserstoffbrücken oder Dipol-Dipol-Wechselwirkungen miteinander assoziiert sind. In der Regel sind in Aggregaten die Primärteilchen flächig miteinander verbunden, in Agglomeraten bestehen in der Regel eher punktförmige Verbindungen, d. h. die Berührungsflächen sind vergleichsweise klein.

Die aus den Primärteilchen gebildeten Aggregate und/oder Agglomerate sind deutlich größer als die Primärteilchen, so dass das Fließverhalten einer organischen Matrix mit solchen Füllstoffen erheblich beeinflusst bzw. verschlechtert wird.

Die Erfindung hat nun erkannt, dass man ausgehend von Füllstoffen, bei denen nanoskalige Primärpartikel agglomeriert und/oder aggregiert sind, dennoch zu einem Dentalmaterial mit guten mechanischen Eigenschaften kommen kann. Diese aggregierten bzw. agglomerierten Füllstoffe werden zunächst organisch oberflächenmodifiziert und anschließend in das organische Bindemittel einarbeiten, wobei durch das Einarbeiten Agglomerate und Aggregate soweit zerstört werden, dass mindestens 50 Gew.-% der Nanopartikel einen Teilchendurchmesser von kleiner als 200 nm (bestimmt nach dem unten näher erläuterten Verfahren) aufweisen. Die Erfindung erlaubt somit die Verwendung kostengünstiger Ausgangsmaterialien für den nanoskaligen Füllstoff und erreicht dennoch, dass die erfindungsgemäßen Dentalmaterialien, die durch nanoskalige Füllstoffe verliehenen vorteilhaften Eigenschaften (insbesondere mechanischen Eigenschaften) aufweisen.

Erfindungsgemäß ist der nanoskalige Füllstoff gemäß Merkmal b) noch teilaggregiert, d. h. eine im Anspruch näher definierte Mindestmenge der Nanopartikel sind aggregierte Teilchen, bei denen zwei oder mehr Primärteilchen und/oder Agglomerate durch starke Kräfte verbunden sind. Bei sehr kleinen Primärteilchen können auch die Aggregate noch einen Teilchendurchmesser von kleiner als 100 nm aufweisen, häufig werden jedoch solche Aggregate die angegebene Grenze des Teilchendurchmessers von 100 nm überschreiten.

Die Erfindung hat somit erkannt, dass eine Teilaggregierung der Primärpartikel des Nanofüllstoffes die vorteilhaften Eigenschaften eines nanoskaligen Füllstoffes nicht oder nur unwesentlich beeinträchtigt und dass auf die aufwendige Herstellung von lediglich aus Primärteilchen bestehenden Nanofüllstoffen (bspw. durch das Sol-Gel-Verfahren) verzichtet werden kann.

Füllstoffe wie beispielsweise flammenpyrolytisch gewonnenes Siliziumdioxid besitzen nanoskalige Primärpartikel, die sowohl durch starke Aggregatkräfte (insbesondere Sinterbindungen) als auch schwache Agglomeratkräfte in größeren Aggregaten und/oder Agglomeraten zusammengehalten werden. Dem erfindungsgemäßen Verfahren liegt die Erkenntnis zu Grunde, dass sich durch mechanisches Einarbeiten solcher Füllstoffe in ein organisches Bindemittel die Agglomeratbindungen weitgehend und die Aggregatbindungen teilweise lösen, so dass nach dem Einarbeiten der Füllstoff die in Merkmal b) des Anspruchs definierten Parameter aufweist. Die organische Oberflächenmodifikation gemäß Merkmal b) des Anspruchs 1 bewirkt, dass eine erneute Agglomeration von Primärpartikeln bzw. Aggregaten/ Agglomeraten nach dem Einarbeiten in das organische Bindemittel unterbleibt. Bei dieser organischen Oberflächenmodifikation kann es sich insbesondere um eine unten näher erläuterte Silanisierung handeln. Vorzugsweise führt die organische Oberflächenmodifizierung Gruppen auf die Oberfläche der nanoskaligen Füllstoffe ein, die mit dem organischen Bindemittel chemisch reagieren können oder eine hohe Affinität zu diesem organischen Bindemittel haben.

Das erfindungsgemäße Dentalmaterial enthält wenigstens einen weiteren Füllstoff gemäß Merkmal c) des Anspruchs 1. Es kann sich dabei um einen gemahlenen Füllstoff oder um einen sphärischen Füllstoff (beispielsweise einen sphärischen Füllstoff wie in DE-PS-32 47 800 beschrieben) mit jeweils näher definierten Korngrößen handeln. Sowohl sphärische als auch gemahlene Füllstoffe besitzen charakteristische Partikelformen, die sich von den teilaggregierten Nanopartikeln gemäß Merkmal b) deutlich unterscheiden. Nanoskalige Primärteilchen können eine ähnliche Partikelform wie sphärische Füllstoffe gemäß Merkmal c) aufweisen, unterscheiden sich jedoch deutlich in der Partikelgröße.

Die Erfindung stellt ein Dentalmaterial zur Verfügung, das trotz kostengünstiger Herstellung gute mechanische Eigenschaften aufweist.

Vorteilhafte Ausführungsformen der Erfindung sind in den Unteransprüchen angegeben. Die Ansprüche 2 bis 4 nennen bevorzugte Gewichtsanteile des organischen Bindemittels, nanoskaligen Füllstoffs bzw. des zusätzlichen Füllstoffs gemäß Merkmal c) des Anspruchs 1.

Die mittlere Primärteilchengröße des erfindungsgemäßen nanoskaligen Füllstoffs liegt zwischen 1 nm und 80 nm, bevorzugt zwischen 4 nm und 60 nm und besonders bevorzugt zwischen 6 nm und 50 nm. Der erfindungsgemäße nanoskalige Füllstoff weist eine BET-Oberfläche (gemäß DIN 66131 bzw. DIN ISO 9277) zwischen 15 m$^2$/g und 600

$m^2/g$, bevorzugt zwischen 30 $m^2/g$ und 500 $m^2/g$ und besonders bevorzugt zwischen 50 $m^2/g$ und 400 $m^2/g$ auf.

Bei den erfindungsgemäß eingesetzten nanoskaligen Füllstoffen handelt es sich bevorzugt um Metall-, Halbmetall- oder Mischmetalloxide, -Silikate, -Nitride, -Sulfate, -Titanate, -Zirkonate, -Stannate, -Wolframate oder um eine Mischung aus diesen Verbindungen. Zur Gruppe der Halbmetalle, deren Eigenschaften (vor allem Aussehen und elektrische Leitfähigkeit) zwischen denen der Metalle und der Nichtmetalle liegen, gehören Bor, Silizium, Germanium, Arsen, Antimon, Bismut, Selen, Tellur und Polonium (vgl. Römpp Chemie Lexikon, Georg Thieme Verlag, 1990, S. 1711). Die Gruppe der Metalle ist im Periodensystem links von der Gruppe der Halbmetalle zu finden, d.h. dazu gehören die Hauptgruppenmetalle, Nebengruppenmetalle, Lanthanide und Actinide. Und unter dem Begriff Mischmetalloxid, -nitrid, usw. ist hier eine chemische Verbindung zu verstehen, in der mindestens zwei Metalle und/oder Halbmetalle zusammen mit dem entsprechenden Nichtmetallanion (Oxid, Nitrid, usw.) chemisch miteinander verbunden sind.

Bei den erfindungsgemäß eingesetzten nanoskaligen Füllstoffen handelt es sich besonders bevorzugt um Siliziumdioxid, Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

Bei dem erfindungsgemäßen Verfahren werden diese nanoskaligen Füllstoffe dadurch erhalten, dass kommerziell erhältliche, aggregiert-agglomerierte Nanofüllstoffe (wie z. B. pyrogene Kieselsäuren) in einem organischen Lösungsmittel dispergiert und an der Oberfläche organisch modifiziert werden. Bei dieser organischen Modifikation werden funktionelle Gruppen auf die Oberfläche der Nanopartikel aufgebracht, die zum einen entweder kovalent oder adsorbativ an die Nanopartikel gebunden sind und die zum anderen mit dem organischen Bindemittel chemisch reagieren können oder eine hohe Affinität zu dem organischen Bindemittel haben. In den weiteren Schritten wird direkt die Dispersion der modifizierten Nanofüllstoffe in dem Lösungsmittel verwendet oder bevorzugt das Lösungsmittel abgezogen und dann das trockene Nanopulver in das organische Bindemittel eingearbeitet. Die organische Oberflächenmodifizierung kann auch direkt im organischen Bindemittel erfolgen.

Durch diese Verfahrensweise werden überraschenderweise die Agglomerate und teilweise Aggregate dauerhaft soweit zerkleinert, dass mindestens 50 %, bevorzugt mindestens 60 % und besonders bevorzugt mindestens 80 % der Nanopartikel einen Teilchendurchmesser von kleiner 100 nm aufweisen. Es wird jedoch keine vollständige Deaggregation erreicht, d. h. mindestens 20 %, bevorzugt mindestens 30 %, bevorzugt mindestens 40 % und besonders bevorzugt mindestens 50 % der Nanopartikel sind aggregierte Teilchen.

Die organische Modifikation der Oberfläche der aggregiert-agglomerierten Nanofüllstoffe erfolgt bevorzugt durch Behandeln mit einem Siloxan, Chlorsilan, Silazan, Titanat, Zirkonat, Wolframat oder mit einer organischen Säure (wie sie z. B. in US 6,387,981 beschrieben sind), einem organischen Säurechlorid oder -anhydrid. Die Siloxane, Chlorsilane, Silazane, Titanate, Zirkonate und Wolframate haben besonders bevorzugt die allgemeinen Formeln $Si(OR')_nR_{4-n}$, $Si\,Cl_nR_{4-n}$, $(R_mR''_{3-m}Si)_2NH$, $Ti(OR')_nR_{4-n}$, $Zr(OR')_nR_{4-n}$ und $W(OR')_nR_{6-n}$, wobei m und n 1, 2 oder 3 ist; bevorzugt ist n = 3. Bei der über den Sauerstoff gebundenen Gruppe R' handelt es sich ebenso wie bei R'' um eine beliebige organische funktionelle Gruppe, bevorzugt um eine Alkylgruppe und besonders bevorzugt um eine Methyl-, Ethyl-, Propyl- oder Isopropylgruppe. Die funktionelle Gruppe R ist eine beliebige organische Gruppe und direkt über ein Kohlenstoffatom an das Silizium, Titan, Zirkon oder Wolfram gebunden. Wenn m oder n 1 oder 2 ist, können die Gruppen R gleich oder verschieden sein. R wird bevorzugt so ausgewählt, dass es eine oder mehrere funktionelle Gruppen besitzt, die mit dem organischen Bindemittel chemisch reagieren können oder eine hohe Affinität zu dem organischen Bindemittel haben. Diese funktionellen Gruppen sind auch bei den ebenfalls zur organischen Oberflächenmodifikation einsetzbaren, oben aufgeführten organischen Säuren, Säurechloriden und -anhydriden enthalten. Es handelt sich bevorzugt um Acrylat-, Methacrylat-, Cyanacrylat-, Acrylamid-, Methacrylamid-, Vinyl-, Allyl-, Epoxid-, Oxetan-, Vinylether-, Amino-, Säure-, Säureester-, Säurechlorid-, Phosphat-, Phosphonat-, Phosphit-, Thiol-, Alkohol- und/oder Isocyanatgruppen. Die bei der organischen Oberflächenmodifikation der Nanofüllstoffe gebildeten Nebenprodukte wie z. B. Alkohole, Salzsäure oder Ammoniak werden in den Folgeschritten bis auf mögliche Reste (Verunreinigungen) entfernt, d. h. sie sind in dem später hergestellten Dentalmaterial nicht mehr oder nur in geringen Mengen von ≤ 0,1 Gew.-%, vorzugsweise ≤ 0,05 Gew.-% enthalten.

Eine weitere vorteilhafte Option der Erfindung ist die organische Modifikation der Oberfläche der Nanofüllstoffe mit Farbstoffen. In diesem Fall beinhaltet die Gruppe R der zur Oberflächenmodifikation verwendeten Verbindung einen Farbstoff oder eine funktionelle Gruppe, die mit einem Farbstoff reagieren kann. Die Anbindung des Farbstoffs an die Oberfläche der Nanofüllstoffe kann sowohl über eine kovalente Bindung als auch über eine ionische Bindung erfolgen.

Bei dem Lösungsmittel, in dem die organische Oberflächenmodifikation der Nanofüllstoffe durchgeführt wird, handelt es sich bevorzugt um ein polar aprotisches Lösungsmittel und besonders bevorzugt um Aceton, Butanon, Essigsäureethylester, Methylisobutylketon, Tetrahydrofuran oder Diisopropylether. Weiterhin ist die direkte organische Oberflächenmodifikation der Nanofüllstoffe in dem zur Herstellung der Dentalmaterialien zu verwendendem organischen Bindemittel eine besonders bevorzugte Verfahrensweise. In diesem Fall ist das organische Bindemittel das zu verwendende Lösungsmittel. Zur Beschleunigung der organischen Oberflächenmodifikation der Nanofüllstoffe kann eine Säure als Katalysator zugegeben werden. In jedem Fall müssen katalytische Mengen Wasser, bevorzugt zwischen 0,01 % und 5 %, anwesend sein, um die Modifikation durchzuführen. Dieses Wasser ist an den Oberflächen der als Ausgangssubstanz verwendeten aggregiert-agglomerierten Nanofüllstoffe oftmals bereits als Adsorbat vorhanden. Zur Unterstützung der Reaktion kann weiteres Wasser, z. B. auch in Form einer verdünnten Säure zugegeben werden.

Um den Zerfall der Agglomerate und Aggregate bei der organischen Oberflächenmodifikation in dem organischen Lösungsmittel zu beschleunigen, kann vor oder während der Modifizierung ein zusätzlicher Energieeintrag mit den üblichen Methoden erfolgen. Dies kann z. B. durch einen Hochgeschwindigkeitsrührer, einen Dissolver, eine Perlmühle oder einen Mischer erfolgen. Bei der Verwendung höherviskoser Lösungsmittel ist dies die bevorzugte Vorgehensweise, also besonders dann, wenn das organische Bindemittel direkt als Lösungsmittel verwendet wird. Wenn das organische Bindemittel nicht als Lösungsmittel verwendet wird, kann das zu verwendende organische Bindemittel direkt mit der Dispersion des organisch modifizierten Nanofüllstoffs in dem organischen Lösungsmittel gefüllt werden. In diesem Fall wird das Lösungsmittel nach der Herstellung der Mischung aus organischem Bindemittel und organisch modifiziertem Nanofüllstoff abgezogen oder erst bei der späteren Anwendung des nanogefüllten Dentalmaterials. Letzteres ist besonders bei lösemittelhaltigen Lacken auf der Basis der erfindungsgemäßen Dentalmaterialien eine praktikable Vorgehensweise. Bevorzugt wird der organisch modifizierte Nanofüllstoff von dem Lösungsmittel befreit und als trockenes Pulver weiter verarbeitet. In diesem Fall wird dann das trockene organisch modifizierte Nanopulver zu dem organischen Bindemittel gegeben und unter mechanischem Energieeintrag eingearbeitet. Die Einarbeitung kann z. B. durch einen Hochgeschwindigkeitsrührer, einen Dissolver, eine Perlmühle, einen Walzenstuhl, einen Kneter oder einen Mischer erfolgen.

Bei der Verwendung höherviskoser Lösungsmittel und insbesondere bei der direkten Verwendung des organischen Bindemittels als Lösungsmittel kann es passieren, dass eventuelle Überschüsse und/oder nicht umgesetzte Teile der zur organischen Oberflächenmodifikation der Nanofüllstoffe verwendeten Verbindung nicht aus der Dispersion entfernt werden können. In diesem Fall ist es die bevorzugte Vorgehensweise, dass diese eventuellen Überschüsse und/oder nicht umgesetzten Teile der zur organischen Oberflächenmodifikation der Nanofüllstoffe verwendeten Verbindung durch Reaktion mit einem geeigneten Agens in Stoffe umgesetzt werden, die dann entweder aus der Dispersion entfernt werden oder aber in der Dispersion verbleiben können, wenn sie für den Menschen unbedenklich, d.h. nicht schädigend sind. Eine besonders bevorzugte Vorgehensweise ist die Verwendung von Wasser als Agens, das mit eventuellen Überschüssen und/oder nicht umgesetzten Teilen der zur organischen Oberflächenmodifikation der Nanofüllstoffe verwendeten Verbindung umgesetzt wird.

Als organisches Bindemittel wird eine Verbindung oder eine Mischung mehrerer Verbindungen eingesetzt, die radikalisch und/oder kationisch und/oder anionisch polymerisierbare Gruppen und/oder Gruppen, die eine Aushärtung über eine Kondensations- und/oder Additionsreaktion und/oder über eine Säure-Base-Reaktion erlauben, enthält. Die Verbindungen bestehen aus einem Phospazen-basierten, Silizium-basierten oder organischen (Kohlenstoff-basierten) Grundgerüst und mindestens einer funktionellen Gruppe; die an dieses Grundgerüst gebunden ist und die eine über eine radikalische und/oder kationische und/oder anionische Polymerisationsreaktion und/oder über eine Kondensations- und/oder Additionsreaktion und/oder über eine Säure-Base-Reaktion ablaufende Aushärtung erlaubt. Bei diesen funktionellen Gruppen handelt es sich bevorzugt um Acrylat-, Methacrylat-, Cyanacrylat-, Acrylamid-, Methacrylamid-, Vinyl-, Allyl-, Epoxid-, Oxetan-, Vinylether-, Amino-, Säure-, Säureester-, Säurechlorid-, Phosphat-, Phosphonat-, Phosphit-, Thiol-, Alkohol- und/oder Isocyanatgruppen. Die Säure-, Säureester- und Säurechloridgruppen können z. B. von Carbonsäuren, Phosphorsäuren, Phosphonsäuren oder Sulfonsäuren abgeleitet sein.

Das Grundgerüst kann linear, verzweigt, zyklisch, dendritisch und/oder hyperverzweigt ("hyperbranched") aufgebaut sein. Beim Grundgerüst kann es sich um eine monomere, oligomere oder polymere Struktur handeln. Die chemische Struktur des Grundgerüsts ist aus aliphatischen, zykloaliphatischen, heterozyklischen, aromatischen und/oder heteroaromatischen Segmenten aufgebaut. Innerhalb der aliphatischen, zykloaliphatischen, heterozyklischen, aromatischen und/oder heteroaromatischen Segmente können eine oder mehrere funktionelle Gruppen enthalten sein, z. B. -O-, -S-, -SO-, -SO$_2$-, -NR$^1$-, -PR$^1$-, -P(OR$^1$)-, -POR$^1$-, - PO(OR$^1$)-, -O-PO(OR$^1$)-O-, -CO-, -CO$_2$-, O-CO-O-, -COS-, -CS$_2$-, -C=N-, -N=C=N-, -CO(NR$^1$)-, O-CO-NR$^1$-, -NR$^1$-CO-NR$^2$-, -SiR$^1$R$^2$- und/oder -SiR$^1$R$^2$-O-, wobei R$^1$ =H oder jedbeliebiger organische Rest, bevorzugt ein unsubstituierter oder substituierter

Alkyl- bzw. Aryl-Rest

R$^2$ =H oder jedbeliebiger organische Rest, bevorzugt ein unsubstituierter oder substituierter

Alkyl- bzw. Aryl-Rest; gleich oder verschieden von R$^1$.

Sind aliphatische Segmente im Grundgerüst enthalten, so sind diese bevorzugt von substituierten (z. B. halogenierten) und unsubstituierten Alkyl- und Alkenyl-Verbindungen, Ethern, Estern, Carbonaten, Urethanen, Polyethern, Polyestern, Polycarbonaten und Polyurethanen abgeleitet.

Sind zykloaliphatische Segmente im Grundgerüst enthalten, so sind diese bevorzugt von substituierten (z. B. halogenierten) und unsubstituierten Cycloalkanen (wie z. B. Cyclohexan und seine Derivate), Spiranen (wie z. B. Spiro [3.3]heptan) und bi- und polyzyklischen Kohlenwasserstoffen (wie z.B. Decalin, Norbornan, Norcaran, Pinan, Adamantan, Twistan und Diamantan) abgeleitet.

Sind heterozyklische Segmente im Grundgerüst enthalten, so sind diese bevorzugt von substituierten (z. B. halogenierten) und unsubstituierten Cyclodextrinen, Morpholinen und Iminooxadiazindionen abgeleitet.

Sind aromatische Segmente im Grundgerüst enthalten, so sind diese bevorzugt von substituierten (z. B. halogenierten) und unsubstituierten Benzenen (wie z.B. Benzol, Toluol, Phenol, Anilin und Biphenyl) und kondensierten aromatischen Ringsystemen (wie z. B. Inden, Fluoren, Naphthalin, Acenaphthen, Anthracen, Phenanthren, Naphthacen, Pyren und Chrysen) abgeleitet.

Sind heteroaromatische Segmente im Grundgerüst enthalten, so sind diese bevorzugt von substituierten (z. B. halogenierten) und unsubstituierten Pyrrolen, Furanen, Thiophenen, Indolen, Benzofuranen, Benzothiophenen, Dibenzofuranen, Dibenzothiophenen, Pyrazolen, Imidazolen, Pyridinen, Pyranen, Thiopyranen und Chinolinen abgeleitet.

Als organisches Bindemittel kann auch eine flüssigkristalline Verbindung oder eine Mischung mehrerer Verbindungen, von denen mindestens eine flüssigkristallin ist, eingesetzt werden, die radikalisch und/oder kationisch und/oder anionisch polymerisierbare Gruppen und/oder Gruppen, die eine Aushärtung über eine Kondensations- und/oder Additionsreaktion und/oder über eine Säure-Base-Reaktion erlauben, enthält.

Eine weitere bevorzugte Option ist die Verwendung einer fluoridionenfreisetzenden Verbindung oder einer Mischung mehrerer Verbindungen, von denen mindestens eine Fluoridionen freisetzen kann, als organisches Bindemittel, das zusätzlich radikalisch und/oder kationisch und/oder anionisch polymerisierbare Gruppen und/oder Gruppen, die eine Aushärtung über eine Kondensations- und/oder Additionsreaktion und/oder über eine Säure-Base-Reaktion erlauben, enthält.

Als organische Bindemittel werden besonders bevorzugt Acrylate, Methacrylate, Acrylamide, Methacrylamide, Vinylether, Epoxide, Oxetane, Spiroorthocarbonate, Spiroorthoester, bizyklische Orthoester, bizyklische Monolactone, bizyklische Bislactone, zyklische Carbonate, zyklische Acetale, Allylsulfide, Vinylzyklopropane, organische Phosphate, organische Phosponate, organische Phosphite oder eine Kombination aus diesen Verbindungen eingesetzt. Die Allgemeinheit nicht einschränkend sind nachfolgend einige Beispiele genannt: Methyl(meth)acrylat, Ethyl(meth)acrylat, n- oder i-Propyl(meth)acrylat, n-, i- oder tert.-Butyl(meth)acrylat, Hexyl(meth)acrylat, 2-Ethylhexyl(meth)acrylat, Cyclohexyl(meth)acrylat, Isobornyl(meth)acrylat, Hydroxyethyl(meth)acrylat, Hydroxypropyl(meth)acrylat, Phosphorsäureester von Hydroxyethyl(meth)acrylat bzw. Hydroxypropyl(meth)acrylat, (Meth)acrylsäure, Malonsäure-mono-(meth)acrylatester, Bersteinsäure-mono-(meth)acrylatester, Maleinsäure-mono-(meth)acrylatester, Glycerin(meth)acrylat, Glycerin(meth)acrylatester, Glycerindi(meth)acrylat, Glycerindi(meth)acrylatester (wie z. B. Glycerindi(meth)acrylatsuccinat), 4-(Meth)acryloyloxyethyltrimellithsäure, Bis-4,6- bzw. Bis-2,5-(Meth)acryloyloxyethyltrimellithsäure, 2-(((Alkylamino)-carbonyl)oxy)ethyl-(meth)acrylate, Allyl(meth)acrylat, Butandioldi(meth)acrylat, Hexandioldi(meth)acrylat, Decandioldi(meth)acrylat, Dodecandioldi(meth)acrylat, Ethylenglykoldi(meth)acrylat, Diethylenglykoldi(meth)acrylat, Triethylenglykoldi(meth)acrylat, Tetraethylenglykoldi(meth)acrylat, Polyethylenglykoldi(meth)acrylate, Glycerindi(meth)acrylat, Glycerolpropoxytri(meth)acrylat, Trimethylolpropantri(meth)acrylat, ethoxylierte und/oder propoxylierte Trimethylolpropantri(meth)acrylate, Pentaerythrittetra(meth)acrylat, Dipentaerythritolhexa(meth)acrylat, Bisphenol-A-di(meth)acrylat, ethoxylierte und/oder propoxylierte Bisphenol-A-di(meth)acrylate, 2,2-Bis-4-(3-(meth)acryloxy-2-hydroxy-propoxy)-phenylpropan und davon abgeleitete Verbindungen, Chlor- und Bromphosphorsäureester des Bisphenol-A-glycidyl(meth)acrylats, Urethan(meth)acrylate (wie z. B. 7,7,9-Trimethyl-4,13-dioxo-3,14-dioxa-5,12-diazahexadecan-1,16-dioxy-dimethacrylat), Polyesterurethan(meth)acrylate, Polyester(meth)acrylate, Polycarbonat(meth)acrylate, Polyamid(meth)acrylate, Polyimid(meth)acrylate, Phosphazen(meth)acrylate und Siloxan(meth)acrylate; Ethylvinylether, n- oder i-Propylvinylether, n-, i- oder tert.-Butylvinylether, Hexylvinylether, Octylvinylether, Cyclohexylvinylether, Cyclohexyl-3,4-epoxy-1-methylvinylether, Dimethanol-cyclohexyl-monovinylether, 1,4-Dimethanolcyclohexyl-divinylether, Propandioldivinylether, Butandioldivinylether, Hexandioldivinylether, Octandioldivinylether, Decandiolvinylether, Ethylenglykoldivinylether, Diethylenglykoldivinylether, Triethylenglykoldivinylether, Triethylenglykol-monovinylether-mono(meth)acrylsäureester, Polyethylenglykoldivinylether, Tripropylenglykoldivinylether, Glycerintrivinylether, Pentaerythrittetravinylether, 7,7,9-Trimethyl-4,13-dioxo-3,14-dioxa-5,12-diazahexadecan-1,16-dioxydivinylether, Bisphenol-A-divinylether, ethoxylierte und/oder propoxylierte Bisphenol-A-divinylether , Polyestervinylether, Polycarbonatvinylether, Polyacrylatvinylether, Polyamidvinylether, Polyimidvinylether, Polyurethanvinylether, Phosphazenvinylether und Siloxanvinylether; Alkylglycidylether, Glycidol, Glycidyl(meth)acrylat, Dipentendioxid, 1,2-Epoxyhexadecan, Bis-(3,4-epoxycyclohexyl)-adipat, Vinylcyclohexenoxide, Vinylcyclohexendioxide, Epoxycyclohexancarboxylate (wie z. B. 3,4-Epoxycyclohexylmethyl-3,4-epoxycyclohexen-carboxylat), Butandioldiglycidylether, Hexandioldiglycidylether, Dodecandioldiglycidylether, Diglycidylether der Polyethylenglykole und der Polypropylenglykole, Diglycidylether von substituierten (z. B. halogenierten) und unsubstituierten Bisphenolen (wie z. B. Bisphenol-A, Bisphenol-C und Bisphenol-F), Resorcindiglycidylether, Trimetylolethantriglycidylether, Trimetylolpropantriglycidylether, Polybutadienpolyepoxide, Polyesterepoxide, Polycarbonatepoxide, Polyacrylatepoxide, Polyamidepoxide, Polyimidepoxide, Polyurethanepoxide, Phosphazenepoxide und Siloxanepoxide; 3,3-disubstituierte Oxetane und Dioxetane (wie z.B. 3-Ethyl-3-(2-hydroxyethyl)-oxetan); (trans/trans)-2,3,8,9-Di(tetramethylen)-1,5,7,11-tetraoxaspira-[5.5]-undecan; substituierte 1,3-Dioxolane (wie z. B. 2-Phenyl-4-methylen-1,3-dioxolan); difunktionelle 6-Methylen-1,4-dithiepane sowie die Umsetzungsprodukte von nukleophilen (Meth)acrylaten wie z. B. 2-Hydroxyethyl(meth)acrylat oder Glycerin(meth)acrylatestern mit reaktiven Phosphorsäure-, Phosphonsäure- oder Phosphinsäurederivaten wie z. B. $P_2O_5$, $POCl_3$ oder $PCl_3$.

Die erhaltenen Mischungen aus organischem Bindemittel und organisch modifiziertem Nanofüllstoff zeichnen sich durch eine hohe Transparenz und eine geringe Viskosität aus. Bei der Verwendung als Dentalmaterial sind sie allerdings nicht für alle Anwendungen geeignet, da sie einen hohen Polymerisationsschrumpf und eine relativ geringe mechanische Festigkeit aufweisen. Daher wird erfindungsgemäß wenigstens ein anorganischer und/oder organischer Füllstoff ausgewählt aus der Gruppe bestehend aus gemahlenen Füllstoffen mit einer mittleren Korngröße zwischen 0,2 μm und 50 μm und sphärischen Füllstoffen mit einer mittleren Korngröße zwischen 0,1 μm und 50 μm in die oben beschriebenen Mischungen homogen eingearbeitet. Dadurch wird ein Material erhalten, dass sich durch eine höhere Druckfestigkeit, einen geringeren Polymerisationsschrumpf und eine verbesserte Abriebfestigkeit auszeichnet.

Diese erfindungsgemäßen Dentalmaterialien können unterschiedlichste Konsistenzen aufweisen; sie können z. B. fließfähig als auch standfest, d. h. modellierbar sein. Zudem können sie verschiedenste rheologische Eigenschaften aufweisen; sie können z. B. thixotrop, dilatant oder struktur-viskos sein. Eine hohe Transluzenz, d. h. eine geringe Opazität bei den erfindungsgemäßen Dentalmaterialien ist ebenfalls möglich.

Der oben beschriebene anorganische und/oder organische Füllstoff wird unter mechanischem Energieeintrag in die Mischung aus organischem Bindemittel und organisch modifiziertem Nanofüllstoff eingearbeitet. Die Einarbeitung kann z. B. durch einen Hochgeschwindigkeitsrührer, einen Dissolver, eine Perlmühle, einen Walzenstuhl, einen Kneter oder einen Mischer erfolgen.

Bei dem erfindungsgemäßen Verfahren erfolgt die Oberflächenmodifikation des nanoskaligen Füllstoffs entweder vor oder zeitgleich mit dem Einarbeiten in das organische Bindemittel. Der weitere (sphärische und/oder gemahlene) Füllstoff kann vor, gleichzeitig mit oder nach dem nanoskaligen Füllstoff in das organische Bindemittel eingearbeitet werden.

Der anorganische und/oder organische Füllstoff kann ein nichtreaktiver Füllstoff, ein reaktiver Füllstoff oder eine Mischung aus diesen beiden Füllstoff-Typen sein. Unter einem reaktiven Füllstoff wird hier ein Füllstoff verstanden, der unter Zutritt von Wasser Ionen freisetzt und somit zu einer Aushärtung des Materials über eine Säure-Base-Reaktion führen kann. Diese reaktiven Füllstoffe werden z.B. zur Herstellung von Compomeren und Glasionomerzementen verwendet und sind z. B. in D.C. Smith, Biomaterials 19, S. 467-478 (1998) beschrieben.

Als anorganischer und/oder organischer Füllstoff werden bevorzugt Quarzpulver, Glaspulver, Glaskeramikpulver, Metalloxide, Metallhydroxide, gefüllte und/oder ungefüllte Splitterpolymerisate, gefüllte und/oder ungefüllte Perlpolymerisate, sphärische Füllstoffe wie z. B. in der DE-PS 3247800 beschrieben oder eine Mischung aus diesen Füllstoffen eingesetzt. Bei den Splitter- und Perlpolymerisaten handelt es sich um Homo- oder Copolymere der schon beschriebenen (als organisches Bindemittel verwendbaren) polymerisierbaren Verbindungen, wobei diese Homo- oder Copolymere mit den beschriebenen Nanofüllstoffen und/oder anorganischen Füllstoffen wie z. B. Quarzpulver, Glaspulver, Glaskeramikpulver, pyrogenen oder nass gefällten Kieselsäuren gefüllt sein können. Splitterpolymerisate werden durch Mahlung der entsprechenden Polymerisationsprodukte erhalten.

Als anorganischer und/oder organischer Füllstoff werden besonders bevorzugt Bariumsilikatgläser, Strontiumsilikatgläser, Borataluminosilikatgläser, Phosphataluminosilikatgläser, Fluoroaluminosilikatgläser, Calciumsilikate, Zirkonsilikate, Natriumaluminiumsilikate, Schichtsilikate, Bentonite, Zeolithe einschließlich der Molekularsiebe, die Oxide sowie die Hydroxide der Alkali- und der Erdalkalimetalle, Apatit, gefüllte Splitterpolymerisate, sphärische Füllstoffe wie z. B. in der DE-PS 3247800 beschrieben oder eine Mischung aus diesen Füllstoffen eingesetzt.

Eine bevorzugte Variante ist die Verwendung eines anorganischen und/oder organischen Füllstoffs, bei dem durch organische Modifikation funktionelle Gruppen auf die Oberfläche des Füllstoffs aufgebracht werden, die mit dem organischen Bindemittel chemisch reagieren können oder eine hohe Affinität zu dem organischen Bindemittel haben. Bei diesen funktionellen Gruppen handelt sich bevorzugt um Acrylat-, Methacrylat-, Cyanacrylat-, Acrylamid-, Methacrylamid-, Vinyl-, Allyl-, Epoxid-, Oxetan-, Vinylether-, Amino-, Säure-, Säureester-, Säurechlorid-, Phosphat-, Phosphonat-, Phosphit-, Thiol-, Alkohol- und/oder Isocyanatgruppen. Bevorzugt werden diese Gruppen über die schon bei der Oberflächenmodifikation der Nanofüllstoffe beschriebenen Verbindungen (Siloxane, Chlorsilane, Silazane, Titanate, Zirkonate, Wolframate, organische Säuren, organische Säurechloride oder -anhydride) eingeführt.

Eine besonders bevorzugte Variante ist die Verwendung eines anorganischen und/oder organischen Füllstoffs, der röntgenopak ist und in solchen Mengen in das erfindungsgemäße Dentalmaterial eingearbeitet wird, dass das erfindungsgemäße Dentalmaterial eine Röntgenopazität (gemäß ISO 4049-2000) bevorzugt ≥ 100 % Al und besonders bevorzugt ≥ 200 % Al aufweist.

Zur Einstellung der Viskosität des erfindungsgemäßen Dentalmaterials kann optional zusätzlich pyrogene oder nass gefällte Kieselsäure in das erfindungsgemäße Dentalmaterial eingearbeitet werden.

Bei dem erfindungsgemäßen Dentalmaterial kann es sich sowohl um ein Ein-Komponenten-Material als auch um ein MehrKomponenten-Material handeln, wobei im letzteren Fall mindestens eine der Komponenten, bevorzugt alle Komponenten der erfindungsgemäßen Zusammensetzung entsprechen. Gegenstand der Erfindung ist somit auch ein Kit zur Herstellung eines erfindungsgemäßen Dentalmaterials, das Kit kann ein oder mehrere Komponenten enthalten.

Bei dem Mehrkomponentenkit oder -system erfolgt die Herstellung eines erfindungsgemäßen Dentalmaterials durch Anmischen der Komponenten im vorgegebenen Mischungsverhältnis und anschließendem Aushärten.

Zur Aushärtung des erfindungsgemäßen Dentalmaterials wird bevorzugt ein Initiator bzw. mehrere Initiatoren und optional ein Coinitiator bzw. mehrere Coinitiatoren in das erfindungsgemäße Dentalmaterial eingearbeitet. Dabei können Initiator bzw. Initiatoren und Coinitiator bzw. Coinitiatoren zusammen in einer Komponente und/oder getrennt in zwei oder mehreren Komponenten enthalten sein. Das erfindungsgemäße Dentalmaterial kann somit thermisch, chemisch, photochemisch, d. h. durch Bestrahlung mit UV- und/oder sichtbarem Licht, und/oder durch Reaktion mit der Mund- und/oder Luftfeuchte ausgehärtet werden.

Die hier verwendbaren Initiatoren können z. B. Photoinitiatoren sein. Diese sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm und optional durch die zusätzliche Reaktion mit einem oder mehreren Coinitiatoren die Aushärtung des Materials bewirken können. Bevorzugt werden hier Phosphinoxide, Bezoinether, Benzilketale, Acetophenone, Benzophenone, Thioxanthone, Bisimidazole, Metallocene, Fluorone, $\alpha$-Dicarbonylverbindungen, Aryldiazoniumsalze, Arylsulfoniumsalze, Aryliodoniumsalze, Ferroceniumsalze, Phenylphosphoniumsalze oder eine Mischung aus diesen Verbindungen eingesetzt.

Besonders bevorzugt werden Diphenyl-2,4,6-trimethylbenzoyl-phosphinoxid, Benzoin, Benzoinalkylether, Benzildialkylketale, $\alpha$-Hydroxy-acetophenon, Dialkoxyacetophenone, $\alpha$-Aminoacetophenone, i-Propylthioxanthon, Campherchinon, Phenylpropandion, 5,7-Diiodo-3-butoxy-6-fluoron, (eta-6-Cumol)(eta-5-cyclopentadienyl)eisen-hexafluorophosphat, (eta-6-Cumol)(eta-5-cyclopentadienyl)eisentetrafluoroborat, (eta-6-Cumol)(eta-5-cyclopentadienyl)eisen-hexafluoroantimonat, substituierte Diaryliodoniumsalze, Triarylsulfoniumsalze oder eine Mischung aus diesen Verbindungen eingesetzt.

Als Coinitiatoren für eine photochemische Aushärtung werden bevorzugt tertiäre Amine, Borate, organische Phosphite, Diaryliodoniumverbindungen, Thioxanthone, Xanthen, Fluorene, Fluorone, $\alpha$-Dicarbonylverbindungen, kondensierte Polyaromaten oder eine Mischung aus diesen Verbindungen eingesetzt. Besonders bevorzugt werden N,N-Dimethyl-p-toluolidin, N,N-Dialkyl-alkyl-aniline, N,N-Dihydroxyethyl-p-toluidin, 2-Ethylhexyl-p-(dimethyl-amino)-benzoat, Butyrylcholin-triphenylbutyl-borat oder eine Mischung aus diesen Verbindungen eingesetzt.

Als Initiatoren können auch sogenannte thermische Initiatoren eingesetzt werden, die durch die Aufnahme von thermischer Energie bei erhöhter Temperatur die Aushärtung des Materials bewirken können. Hierbei werden bevorzugt anorganische und/oder organische Peroxide, anorganische und/oder organische Hydroperoxide, $\alpha,\alpha'$-Azo-bis(isobutyroethylester), $\alpha,\alpha'$-Azo-bis(isobutyronitril), Benzpinakole oder eine Mischung aus diesen Verbindungen eingesetzt. Besonders bevorzugt werden Diacylperoxide wie z. B. Benzoylperoxid oder Lauroylperoxid, Cumolhydroperoxid, Benzpinakol, 2,2'-Dimethylbenzpinakol oder eine Mischung aus diesen Verbindungen eingesetzt.

Für eine chemische Aushärtung bei Raumtemperatur wird i. a. ein Redoxinitiatorsystem verwendet, das aus einem bzw. mehreren Initiatoren und einem als Aktivator dienenden Coinitiator bzw. Coinitiatoren besteht. Aus Gründen der Lagerstabilität werden Initiator bzw. Initiatoren und Coinitiator bzw. Coinitiatoren in räumlich voneinander getrennten Teilen des erfindungsgemäßen Dentalmaterials eingearbeitet, d. h. es liegt ein mehrkomponentiges, bevorzugt ein zweikomponentiges Material vor. Als Initiator bzw. Initiatoren werden bevorzugt anorganische und/oder organische Peroxide, anorganische und/oder organische Hydroperoxide, Barbitursäurederivate, Malonylsulfamide, Protonensäuren, Lewis- oder Broensted-Säuren bzw. Verbindungen, die solche Säuren freisetzen, Carbeniumionen-Donatoren wie z. B. Methyltriflat oder Triethylperchlorat oder eine Mischung aus diesen Verbindungen und als Coinitiator bzw. als Coinitiatoren bevorzugt tertiäre Amine, Schwermetallverbindungen, insbesondere Verbindungen der 8. und der 9. Gruppe des Periodensystems ("Eisen- und Kupfergruppe"), Verbindungen mit ionogen gebundenen Halogenen oder Pseudohalogenen wie z. B. quartäre Ammoniumhalogenide, schwache Broenstedt-Säuren wie z. B. Alkohole und Wasser oder eine Mischung aus diesen Verbindungen eingesetzt.

In dem erfindungsgemäßen Dentalmaterial kann auch jede denkbare Kombination der oben beschriebenen Initiatoren und Coinitiatoren enthalten sein. Ein Beispiel hierfür sind sogenannte dualhärtende Dentalmaterialien, die sowohl Photoinitiatoren und optional die entsprechenden Coinitiatoren für eine photochemische Aushärtung als auch Initiatoren und entsprechende Coinitiatoren für eine chemische Aushärtung bei Raumtemperatur enthalten.

Das erfindungsgemäße Dentalmaterial kann zur Einstellung bestimmter Eigenschaften zusätzlich sogenannte Additive bzw. Modifikatoren enthalten. Die Allgemeinheit nicht einschränkend sind nachfolgend einige Beispiele genannt: anorganische und/oder organische Farbpigmente bzw. Farbstoffe, Stabilisatoren (wie z. B. substituierte und unsubstituierte Hydroxyaromaten, Tinuvine, Terpinene, Phenothiazin, sogenannte HALS - Hindered Amine Light Stabilizers - und/oder Schwermetallfänger wie EDTA), Weichmacher (wie z. B. Polethylenglykole, Polypropylenglykole, ungesättigte Polyester, Phthalate, Adipate, Sebacate, Phosphorsäureester, Phosphonsäureester und/oder Zitronensäureester), ionenabgebende Substanzen, insbesondere solche die Fluoridionen freisetzen (wie z. B. Natriumfluorid, Kaliumfluorid, Yttriumfluorid, Ytterbiumfluorid und/oder quartäre Ammoniumfluoride), bakterizide oder antibiotisch wirksame Substanzen (wie z. B. Chlorhexidin, Pyridiniumsalze, Penicilline, Tetracycline, Chloramphenicol, antibakterielle Makrolide und/oder Polypeptid-Antibiotika) und/oder Lösungsmittel (wie z. B. Wasser, Aceton, Ethanol, i-Propanol, Butanon und/oder Essigsäureethylester).

Das erfindungsgemäße Dentalmaterial kann für die prothetische, konservierende und präventive Zahnheilkunde verwendet wenden. Ohne Anspruch auf Vollständigkeit seien stellvertretend einige Anwendungsbeispiele genannt: Zahnfüllungsmaterialien, Stumpfaufbaumaterialien, Materialien für provisorische Kronen und Brücken, Zahnzemente, Adhäsive, Materialien für künstliche Zähne, Verblendmaterialien, Versiegelungsmaterialien und Dentallacke.

Die Erfindung wird nachfolgend anhand von Ausführungsbeispielen erläutert. Zunächst werden verschiedene verwendete Mess- und Prüfverfahren erläutert, anschließend folgen die Beispiele und Vergleichsbeispiele.

### I. Mess- und Prüfverfahren

### 1. Teilchengrößenbestimmung der nanoskaligen Füllstoffe

Die Teilchengrößenbestimmungen wurden mittels dynamischer Lichtstreuung (3D-PCS) durchgeführt. Die Methode erlaubt die Bestimmung der Gewichtsanteile von Teilchengrößen im Bereich von 1 nm bis zu einigen Mikrometern. Die obere Grenze der Methode ist dadurch gegeben, dass größere Teilchen in der Messlösung sedimentieren und somit nicht erfasst werden können.

Alle Proben wurden als verdünnte Dispersionen in 2-Butanon untersucht, wobei ein Feststoffanteil von etwa 0,5 Gew-% eingestellt wurde. Diese Verdünnung wurde in erster Linie gewählt, um Teilchen-Teilchen-Wechselwirkungen sicher auszuschließen.

### 2. Teilchengrößestimmung der Mikrofüllstoffe

Die Partikelgrößenverteilung wird mittels der Laserdiffraktometrie (Typ: Coulter LS 130) erfasst. Es wird bei diesem Verfahren der Gewichtsanteil von Partikeln, die eine bestimmte Größe haben, bestimmt. Ein Charakteristikum ist der $d_{50}$-Wert, der angibt, dass die Hälfte (50 %) der Gesamtmasse der Teilchen diese Größe über- bzw. unterschreiten. Die Vermessung der Teilchen erfolgt in verdünnten, zumeist wässrigen Dispersionen.

### 3. Ermittlung des Partikelzahlanteils der Aggregate der nanoskaligen Füllstoffe

Die Dispersion aus Füllstoff und organischen Bindemittel wird mittels eines Elektronenmikroskops untersucht. Bei der Auswertung werden diejenigen Teilchen ausgezählt, bei denen Primärteilchen flächig aneinander gebunden (aggregiert) sind.

### 4. Polymerisationsschrumpf

Der Polymerisationsschrumpf wurde mit der sogenannten Auftriebsmethode bestimmt. Diese Methode arbeitet nach dem Prinzip der hydrostatischen Waage auf der Grundlage des Archimedischen Prinzips: Der Auftrieb, den ein Körper erfährt, ist gleich dem Gewicht der von ihm verdrängten Flüssigkeitsmenge. Damit ist der Auftrieb gleich dem Gewichtsverlust, den er nach Eintauchen in eine Flüssigkeit erfährt.

In destilliertem Wasser bei 4°C ist der Auftrieb und damit der Gewichtsverlust direkt gleich dem Volumen des untersuchten Körpers.

Zur Messung des Polymerisationsschrumpfes, d. h. der Volumenabnahme, wurde in den vorliegenden Fällen die jeweils zu vermessende Probe an einem sehr dünnen Faden befestigt und fest mit der Waageschale einer elektronischen Analysewaage (Sartorius A200 S) verbunden. Es wurde das Trockengewicht $m_{Luft}$ gemessen. Dann wurde zusätzlich ein wassergefülltes Becherglas in die Waage eingebracht, in welches die Probe vollständig eintauchte. Es wurden die Masse der Probe $m_1$ sowie die Wassertemperatur und damit die Dichte des Wassers $\rho_1$ bestimmt. Die Probe wurde anschließend mit einer Espe Elipar II Halogenlampe ausgehärtet (dreimal 40 Sekunden) und für 24 Stunden bei 23°C trocken gelagert. Die Masse der Probe $m_2$ (wiederum vollständig eingetaucht in Wasser) sowie die Wassertemperatur und damit die Dichte des Wassers $\rho_2$ wurden bestimmt.

Der Polymerisationsschrumpf $\Delta V$ wurde nach der folgenden Formel berechnet:

$$\Delta V \ [\%] \ = \ 100 \ [\Delta m_1 \ - \ (\rho_1 \ / \ \rho_2) \ \Delta m_2] \ / \ \Delta m_1$$

In der Gleichung sind: $\Delta m_1 = m_{Luft} - m_1$ und $\Delta m_2 = m_{Luft} - m_2$

**5. Biegefestigkeit**

Mit Hilfe einer Form aus nicht rostendem Stahl wurden Probekörper mit den Abmessungen $(40 \pm 2)$ mm x $(2 \pm 0,1)$ mm x $(2 \pm 0,1)$ mm hergestellt (Aushärtung in einem Dentacolor XS-Lichtofen: 90 Sekunden je Seite). Die Probekörper wurden in destilliertem Wasser für 23 Stunden bei 40°C gelagert und dann für 1 Stunde bei 23°C gelagert. Anschließend wurde mit Hilfe einer Universaltestmaschine von Zwick (Typ Z 010 / TN2A) die Biegefestigkeit gemäß ISO 4049-2000 gemessen:

- konstante Vorschubgeschwindigkeit: 0,8 mm / min
- Berechnung der Biegefestigkeit (BF) nach der folgenden Formel:

$$\text{BF [MPa]} = (3\ F\ l)\ /\ (2\ b\ h^2)$$

wobei

F    die maximale auf den Probekörper ausgeübte Kraft, in Newton;
l    der Abstand zwischen den Auflagen mit einer Genauigkeit von $\pm$ 0,01 mm, in Millimeter;
b    die Breite des Probekörpers unmittelbar vor der Prüfung gemessen; in Millimeter;
h    die Höhe des Probekörpers unmittelbar vor der Prüfung gemessen; in Millimeter; ist.

**6. Druckfestigkeit**

Mit Hilfe einer Form aus nicht rostendem Stahl wurden zylindrische Probekörper mit einer Höhe von $4 \pm 0,02$ mm und einem Durchmesser von $2 \pm 0,01$ mm hergestellt (Aushärtung mit einer Espe Elipar II Halogenlampe: 40 Sekunden je Seite). Die Probekörper wurden in destilliertem Wasser für 23 Stunden bei 40°C gelagert und dann für 1 Stunde bei 23°C gelagert. Anschließend wurde mit Hilfe einer Universaltestmaschine von Zwick (Typ Z 010 / TN2A) die Druckfestigkeit gemessen:

- konstante Vorschubgeschwindigkeit: 1,0 mm / min
- Berechnung der Druckfestigkeit (DF) nach der folgenden Formel:

$$\text{DF [MPa]} = F\ /\ (\pi\ r^2)$$

wobei

F    die maximale auf den Probekörper ausgeübte Kraft, in Newton;
r    der Radius des Probekörpers; in Millimeter; ist.

**7. Diametrale Zugfestigkeit**

Mit Hilfe einer Form aus nicht rostendem Stahl wurden zylindrische Probekörper mit einer Höhe von $3 \pm 0,01$ mm und einem Durchmesser von $6 \pm 0,01$ mm hergestellt (Aushärtung in einem Dentacolor XS-Lichtofen: 90 Sekunden je Seite). Die Probekörper wurden in destilliertem Wasser für 23 Stunden bei 40°C gelagert und dann für 1 Stunde bei 23°C gelagert. Anschließend wurde mit Hilfe einer Universaltestmaschine von Zwick (Typ Z 010 / TN2A) die Diametrale Zugfestigkeit gemäß ADA-Spezifikation No. 27 von 1977 gemessen:

- konstante Vorschubgeschwindigkeit: 1,0 mm / min
- Berechnung der Diametralen Zugfestigkeit (DZ) nach der folgenden Formel:

$$\text{DZ [MPa]} = 2\ F\ /\ (\pi\ d\ h)$$

wobei

F   die maximale auf den Probekörper ausgeübte Kraft, in Newton;
d   der Durchmesser des Probekörpers; in Millimeter;
h   die Höhe des Probekörpers; in Millimeter; ist.

### 8. Barcolhärte

Mit Hilfe einer Form aus nicht rostendem Stahl wurden zylindrische Probekörper mit einer Höhe von 2,5 $\pm$ 0,1 mm und einem Durchmesser von 25 $\pm$ 0,1 mm hergestellt (Aushärtung in einem Dentacolor XS-Lichtofen: 180 Sekunden je Seite). Anschließend wurde die Barcolhärte mit Hilfe eines Barber-Colman Impressors bestimmt. Dabei wurden jeweils mindestens 5 Werte verteilt auf dem Probekörper gemessen und der Mittelwert gebildet.

### 9. Polymerisationstiefe

Die Polymerisationstiefe wurde gemäß ISO 4049-1988 bestimmt. Dabei wurde als Prüfkörperform ein Hohlzylinder mit einer Höhe von 10 mm und einem Durchmesser von 5 mm gewählt. Die jeweilige Probe wurde für 20 Sekunden mit einer Kulzer Translux EC Halogenlampe belichtet.

### 10. Wasseraufnahme

Die Wasseraufnahme wurde gemäß ISO 4049-2000 bestimmt. Dabei wurde als Prüfkörperform ein Hohlzylinder aus Aluminium mit einer Höhe von 1,0 $\pm$ 0,1 mm und einem Innendurchmesser von 15 $\pm$ 0,1 mm gewählt. Die jeweilige Probe wurde 180 Sekunden je Seite in einem Dentacolor XS Lichtofen belichtet.

### 11. Abrasion

Die Abrasion wurde mit der 1986 von DeGee entwickelten Methode der 3-Medien-Abrasion (vgl. A.J. DeGee et al., J. Dent. Res. 65(5), 1986, S. 654-658) bestimmt.

Zur Herstellung der Probekörper wurde eine den Vertiefungen des Probenrades angepasste Form verwendet. Das Material wurde schichtweise (ca. 2 mm pro Schicht) in die Form eingebracht und für jeweils 40 Sekunden mit einer Espe Tri-Light Halogenlampe ausgehärtet. Unmittelbar danach wurden die Proben noch für 90 Sekunden in einem Kuraray CS 110 Lichtofen nachgehärtet. Die verschiedenen Werkstoffproben wurden in zufälliger Anordnung auf das Probenrad der 3-Medien-Abrasions-Maschine aufgeklebt. Zur Gewährleistung einer gleichmäßigen Rundung und Oberfläche wurde das Probenrad im Nassschleifverfahren (1000 Grid) plangeschliffen. Die Lagerung erfolgte unmittelbar nach der Herstellung für mindestens zwei Wochen bei 37°C in destilliertem Wasser.

Alle Materialien wurden vier mal 50.000 Zyklen in der 3-Medien-Abrasions-Maschine entsprechend der beschriebenen Vorgehensweise belastet (*Krämer 1997*). Die profilometrische Vermessung der Abrasionskörper erfolgte mittels eines computer-gesteuerten Perthometers C5D (Fa. Perthen, Göttingen). Die somit erhaltenen 3-D-Daten wurden dann mit Hilfe der Bildverarbeitungsoberfläche XPERT für Windows ausgewertet. Dazu wurde der Abrasionsbereich genau definiert und aus dem abradierten Volumen der mittlere Höhenverlust unterhalb der eingezeichneten Ebene ausgegeben.

### II. Beispiele und Vergleichsbeispiele

Ohne Einschränkung der Allgemeinheit wird die Erfindung nachfolgend anhand von mehreren Beispielen näher erläutert.

Nanofüllstoff A:

Zunächst wurden 150 g Aerosil OX 50 (Degussa AG) in ein Reaktorgefäß gegeben und unter Vakuum und Rühren wurden 220 g Dichlormethan in das Reaktorgefäß eingesaugt. Danach wurde eine Lösung von 7,6 g 3-Methacryloxypropyltrimethoxysilan (ABCR GmbH & Co. KG), 3,3 g destilliertes Wasser und 12 mg Methacrylsäure in 580 g Dichlormethan ebenfalls in das Gefäß eingesaugt. Nach Beendigung dieses Vorgangs wurde das Lösungsmittelgemisch unter Vakuum abdestilliert und man erhielt ein weißes Pulver.

Nanofüllstoff B:

150 g Aerosil 200 (Degussa AG) wurden in einen 2L-Zweihalskolben eingewogen und mit ca. 1000 g 2-Butanon versetzt. Die zunächst breiige Masse wurde solange mit einem KPG-Rührer gerührt, bis sich eine homogene flüssige Suspension bildete. Dann wurden 95,55 g 3-Methacryloxypropyltrimethoxysilan mit einem Tropftrichter zugetropft. Die dünnflüssige Suspension wurde insgesamt 48 Stunden gerührt. Anschließend wurde das 2-Butanon langsam an einem Rotationsverdampfer abgezogen. Nach dem Entfernen des Lösungsmittels blieb ein weißes, lockeres, grobteiliges poröses Pulver zurück, das leicht zerfiel.

Harz A:

In 649,7 g einer 1:1-Mischung aus Bisphenol-A-diglycidylmethacrylat und Triethylenglykol-dimethacrylat wurden 1,97 g Campherchinon, 3,21 g 2-Ethylhexyl-4-(dimethylamino)-benzoat und 65,8 mg 2,6-Bis-tert.-butyl-4-methyl-phenol gelöst.

Volumenschrumpf nach 24 h: 10,7 %

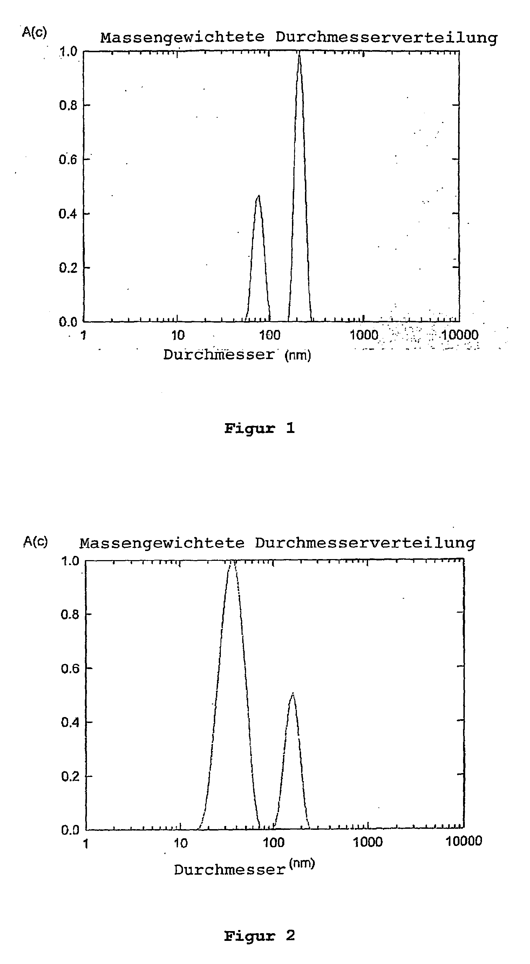

Nanogefülltes Harz B-1 (Vorstufe von Referenzbeispiel 2-1): In 100 g des Harzes A wurden innerhalb von 2 Stunden mit Hilfe eines Dispermaten insgesamt 96,7 g des Nanofüllstoffs A portionsweise eingearbeitet. Auf diese Weise erhielt man ein leicht opakes, mittelviskoses, gefülltes Harz mit einem Füllgrad von 49,2 % an nanoskaligem Siliziumdioxid. Mit Hilfe der dynamischen Lichtstreuung sollte vom nanogefüllten Harz B-1 die Korngrößen-verteilung gemessen werden. Dabei trat jedoch das Problem auf, dass in dem Harz sehr große Aggregate und/oder Agglomerate vorlagen (mit einer mittleren Korngröße $d_{50} > 1\mu$m), die vor bzw. während der Messung separierten und so nicht mitvermessen werden konnten. Daher ist in Abbildung 1 die Korngrößenverteilung nur der Teilchen vom nanogefüllten Harz B-1 abgebildet, die noch vermessen werden konnten.

Transparenz: 56,2 %
Volumenschrumpf nach 24h: 5,2 %

Figur 1 zeigt die Korngrößenverteilung der nicht separierten Teilchen vom nanogefüllten Harz B-1 (dynamische Lichtstreuung)

Peak 1:

Mittelwert = 75,8 nm; Breite = 12,5 %; Amplitude = 34,5 % Peak 2:

Mittelwert = 212,7 nm; Breite = 11,1 %; Amplitude = 65,5 %

Unter Berücksichtigung des relativ hohen Anteils an nicht messbaren aggregierten und/oder agglomerierten Teilchen mit einer Korngröße d > 1 $\mu$m lässt sich aus der obigen Abbildung folgern, dass nur ein sehr geringer Anteil der Teilchen des nanogefüllten Harzes
B-1 eine Korngröße d < 100 nm aufweist.

Nanogefülltes Harz B-2 (Vorstufe von Referenzbeispiel 2-2):

In 100 g des Harzes A wurden innerhalb einer Stunde mit Hilfe eines Labormischers insgesamt 96,7 g des Nanofüllstoffs A portionsweise eingearbeitet. Auf diese Weise erhielt man ein leicht opakes, mittelviskoses, gefülltes Harz mit einem Füllgrad von 49,2 % an nanoskaligem Siliziumdioxid.
Bei diesem Harz konnte die Korngrößenverteilung mit Hilfe der dynamischen Lichtstreuung nicht gemessen werden, da mindestens 80 % des Füllstoffs so stark aggregiert und/oder agglomeriert war, dass die entsprechenden Teilchen so groß waren, dass sie separierten und somit nicht vermessen werden konnten.

Transparenz: 56,4 %
Volumenschrumpf nach 24h: 5,3 %

Nanogefülltes Harz C (Vorstufe von Erfindungsbeispielen):

In 250 g einer 1:1-Mischung aus Bisphenol-A-diglycidylmethacrylat und Triethylenglykoldimethacrylat wurden etwa 50 g des Nanofüllstoffs B gegeben und mit Hilfe eines Dispermaten in 90 Minuten bei 1200 U/min eingearbeitet. Dann wurden weitere 40 g des Nanofüllstoffs B hinzugegeben, und die Mischung 1 Stunde bei 1000 U/min und anschließend über Nacht bei 500 U/min dispergiert. Es wurden insgesamt 225 g des Nanofüllstoffs in Portionen von 30 - 40 g eindispergiert. Anschließend wurden 0,16 Gew.-% Campherchinon, 0,26 Gew.-% 2-Ethylhexyl-4-(dimethylamino)-benzoat und 0,005 Gew.-% 2,6-Bis-tert.-butyl-4-methylphenol in diesem homogenen Gemisch gelöst. Auf diese Weise wurde ein sehr transparentes, mittelviskoses, gefülltes Harz mit einem Füllgrad von 47,2 % an nanoskaligem Siliziumdioxid erhalten.

Im Gegensatz zu den nanogefüllten Harzen B-1 und B-2 trat bei diesem Harz keine Separation auf, so dass in diesem Fall die Korngrößenverteilung aller Teilchen mittels dynamischer Lichtstreuung bestimmt werden konnte (s. Abbildung 2).

Transparenz: 94,5 %
Volumenschrumpf nach 24h: 7,3 %

Figur 2 zeigt eine Korngrößenverteilung vom nanogefüllten Harz C (dynamische Lichtstreuung)

Peak 1:

Mittelwert = 35,2 nm ; Breite = 31,9 %; Amplitude = 77,2 % Peak 2:

Mittelwert = 159,1 nm ; Breite = 17,5 %; Amplitude = 22,8 %

Die obige Abbildung belegt eindeutig, dass mehr als 50 % der Teilchen des nanogefüllten Harzes C eine Korngröße d < 100 nm aufweisen.

In den folgenden Beispielen 1 - 4 werden Hybrid-Composite vorgestellt, die nach dem Stand der Technik (Referenzbeispiele) bzw. gemäß der vorliegenden Erfindung hergestellt wurden. Anzumerken ist, dass alle 5 Pasten sensorisch auf die selbe Konsistenz eingestellt wurden.

Beispiel 1 (Referenz) :

In 34,1 g des Harzes A wurden 6,0 g Aerosil R 974 (Degussa AG) und 130,7 g silanisiertes Barium-Silikatglas mit einer mittleren Korngröße von 1,0 μm eingearbeitet. Anschließend wurde für ca. 60 Minuten bei ca. 200 mbar entgast. Auf diese Weise wurde eine feste, modellierbare, lichthärtende Paste erhalten.

Zusammensetzung: s. Tabelle 1
Eigenschaften: s. Tabelle 2

Beispiel 2-1 (Referenz):

In 50,0 g des nanogefüllten Harzes B-1 wurden zunächst 70,6 g silanisiertes Barium-Silikatglas mit einer mittleren Korngröße von 1,0 μm eingearbeitet. Die entstandene Paste war jedoch so fest und bröselig, dass noch mit 10,0 g des Harzes A verdünnt wurde. Anschließend wurde für ca. 60 Minuten bei ca. 200 mbar entgast. Auf diese Weise wurde eine feste, modellierbare, lichthärtende Paste erhalten.

Zusammensetzung: s. Tabelle 1
Eigenschaften: s. Tabelle 2

Beispiel 2-2 (Referenz):

In 50,0 g des nanogefüllten Harzes B-2 wurden zunächst 60,8 g silanisiertes Barium-Silikatglas mit einer mittleren Korngröße von 1,0 μm eingearbeitet. Die entstandene Paste war jedoch etwas zu fest und wurde daher noch mit 1,0 g des Harzes A verdünnt. Anschließend wurde für ca. 60 Minuten bei ca. 200 mbar entgast. Auf diese Weise wurde eine feste, modellierbare, lichthärtende Paste erhalten.

Zusammensetzung: s. Tabelle 1
Eigenschaften: s. Tabelle 2

Beispiel 3 (Erfindung):

In 50,1 g des nanogefüllten Harzes C wurden 105,5 g silanisiertes Barium-Silikatglas mit einer mittleren Korngröße von 1,0 μm eingearbeitet. Anschließend wurde für ca. 60 Minuten bei ca. 300 mbar entgast. Auf diese Weise wurde eine feste, modellierbare, lichthärtende Paste erhalten.

Zusammensetzung: s. Tabelle 1
Eigenschaften: s. Tabelle 2

Beispiel 4 (Erfindung):

In 54,1 g des gefüllten Harzes C wurden 6,0 g Aerosil R 974 (Degussa AG) und 95,6 g silanisiertes Barium-Silikatglas mit einer mittleren Korngröße von 1,0 μm eingearbeitet. Anschließend wurde für ca. 60 Minuten bei ca. 200 mbar entgast. Auf diese Weise wurde eine feste, modellierbare, lichthärtende Paste erhalten.

Zusammensetzung: s. Tabelle 1
Eigenschaften: s. Tabelle 2

Tabelle 1: Prozentuale Zusammensetzung der Hybrid-Composite (Bsp. 1 - 4)

|  | Bsp. 1 (Referenz) | Bsp. 2-1 (Referenz) | Bsp. 2-2 (Referenz) | Bsp. 3 (Erfindung) | Bsp. 4 (Erfindung) |
|---|---|---|---|---|---|
| Harz A | 20,0 % | 7,65 % | 0,9 % | - | - |
| Nanogefülltes Harz B-1 | - | 38,3 % | - | - | - |
| Nanogefülltes Harz B-2 | - | - | 44,7 % | - | - |
| Nanogefülltes Harz C | - | - | - | 32,2 % | 34,7 % |
| Aerosil R 974 | 3,5 % | - | - | - | 3,9 % |
| Barium-Silikat-glas ($d_{50}$ = 1,0μm) | 76,5 % | 54,05 % | 54,4 % | 67,8 % | 61,4 % |

|  | Bsp. 1 (Referenz) | Bsp. 2-1 (Referenz) | Bsp. 2-2 (Referenz) | Bsp. 3 (Erfindung) | Bsp. 4 (Erfindung) |
|---|---|---|---|---|---|
| Harz A | 20,0 % | 27,1 % | 23,6 % | 17,0 % | 18,3 % |
| Nanofüllstoff A | - | 18,8 % | 22,0 % | - | - |
| Nanofüllstoff B | - | - | - | 15,2 % | 16,4 % |
| Aerosil R 974 | 3,5 % | - | - | - | 3,9 % |
| Barium-Silikat-glas ($d_{50}$ = 1, 0μm) | 76,5 % | 54,1 % | 54,4 % | 67,8 % | 61,4 % |

Tabelle 2: Physikalische und Mechanische Eigenschaften der Hybrid-Composite (Bsp. 1 - 4)

| | Bsp. 1 (Referenz) | Bsp. 2-1 (Referenz) | Bsp. 2-2 (Referenz) | Bsp. 3 (Erfindung) | Bsp. 4 (Erfindung) |
|---|---|---|---|---|---|
| Druckfestigkeit [MPa] | 409 | 431 | 459 | 513 | 493 |
| Volumenschrumpf nach 24h [%] | 3,8 | 4,0 | 4,4 | 3,3 | 3,4 |
| Abrasion [$\mu$m] | 41 | n.b. | n.b. | n.b. | 20 |
| Biegefestigkeit [MPa] | 126 | 131 | 121 | 129 | 125 |
| Diametrale Zugfestigkeit [MPa] | 53 | 59 | 58 | 57 | 57 |
| Barcolhärte | 82 | 82 | 80 | 85 | 79 |
| Polymerisationstiefe [mm] | ≥ 10 | ≥ 10 | ≥ 10 | ≥ 10 | ≥ 10 |
| Wasseraufnahme [$\mu$g/mm$^3$] | 29 | 22 | 22 | 20 | 22 |

In den folgenden Beispielen 5 und 6 werden Mikrofüller-Composite vorgestellt, die nach dem Stand der Technik (Referenzbeispiel) bzw. gemäß der vorliegenden Erfindung hergestellt wurden. Anzumerken ist, dass die beiden Pasten sensorisch auf die selbe Konsistenz eingestellt wurden.

Beispiel 5 (Referenz):

In 40,0 g des Harzes A wurden 14,0 g Aerosil R 974 (Degussa AG) und 86,1 g eines gemahlenen Splitterpolymerisats, das Dodekandioldimethacrylat, Trimethylolpropantrimethacrylat und silanisiertes Aerosil OX 50 enthielt, eingearbeitet. Anschließend wurde für ca. 45 Minuten bei ca. 300 mbar entgast. Auf diese Weise wurde eine feste, modellierbare, lichthärtende Paste erhalten.

Zusammensetzung: s. Tabelle 3
Eigenschaften: s. Tabelle 4

Beispiel 6 (Erfindung):

In 54,0 g des nanogefüllten Harzes C wurden 100,7 g des in Bsp. 5 beschriebenen, gemahlenen Splitterpolymerisats eingearbeitet. Da die entstandene Peste zu fest war, wurde mit 12,4 g des nanogefüllten Harzes C verdünnt. Anschließend wurde für ca. 60 Minuten bei ca. 200 mbar entgast. Auf diese Weise wurde eine feste, modellierbare, lichthärtende Paste erhalten.

Zusammensetzung: s. Tabelle 3
Eigenschaften: s. Tabelle 4

Tabelle 3: Prozentuale Zusammensetzung der Mikrofüller-Composite (Bsp. 5 - 6)

| | Bsp. 5 (Referenz) | Bsp. 6 (Erfindung) |
|---|---|---|
| Harz A | 28,6 % | - |
| Nanogefülltes Harz C | - | 39,7 % |
| Aerosil R 974 | 10,0 % | - |
| Org. Füllstoff (gemahlenes Präpolymerisat) | 61,4 % | 60,3 % |
| | | |

(fortgesetzt)

|  | Bsp. 5 (Referenz) | Bsp. 6 (Erfindung) |
|---|---|---|
| Harz A | 28,6 % | 21,0 % |
| Nanofüllstoff B | - | 18,7 % |
| Aerosil R 974 | 10,0 % | - |
| Org. Füllstoff (gemahlenes Präpolymerisat) | 61,4 % | 60,3 |

Tabelle 4: Physikalische und Mechanische Eigenschaften der Mikrofüller-Composite(Bsp. 5 - 6)

|  | Bsp. 5 (Referenz) | Bsp. 6 (Erfindung) |
|---|---|---|
| Druckfestigkeit [MPa] | 249 | 345 |
| Volumenschrumpf nach 24h [%] | 3,6 | 3,0 |
| Abrasion [$\mu$m] | 64 | 42 |
| Diametrale Zugfestigkeit [MPa] | 36 | 36 |
| Barcolhärte | 59 | 62 |
| Polymerisationstiefe [mm] | $\geq 10$ | $\geq 10$ |
| Wasseraufnahme [$\mu$g/mm$^3$] | 23 | 20 |

Diese Ergebnisse zeigen, dass die Verwendung der erfindungsgemäß eingesetzten Zusammensetzung in Dentalmaterialien zu einer Erhöhung der Druckfestigkeit, zu einer Verringerung des Polymerisationsschrumpfes und zu einer Verbesserung der Abriebfestigkeit führt.

**Patentansprüche**

1.   Gefülltes und polymerisierbares Dentalmaterial, **dadurch gekennzeichnet, dass** es enthält:

a) ein organisches Bindemittel,
b) einen nanoskaligen Füllstoff, der folgende Merkmale aufweist:

- mindestens 50 Gew.-%, bevorzugt mindestens 60 Gew.-% und besonders bevorzugt mindestens 80 Gew.-% der Nanopartikel weisen einen Teilchendurchmesser von kleiner 200 nm, bevorzugt kleiner als 150 nm, besonders bevorzugt kleiner als 100 nm auf,
- mindestens 20 Partikelzahl-%, bevorzugt mindestens 30 Partikelzahl-%, bevorzugt mindestens 40 Partikelzahl-% und besonders bevorzugt mindestens 50 Partikelzahl-% der Nanopartikel sind aggregierte Teilchen,
- der nanoskalige Füllstoff ist organisch oberflächenmodifiziert,

c) wenigstens einen anorganischen und/oder organischen Füllstoff ausgewählt aus der Gruppe bestehend aus einem gemahlenen Füllstoff mit einer mittleren Korngröße zwischen 0,2 $\mu$m und 50 $\mu$m und einem sphärischen Füllstoff mit einer mittleren Korngröße zwischen 0,1 $\mu$m und 50 $\mu$m.

2.   Dentalmaterial nach Anspruch 1, **dadurch gekennzeichnet, dass** es 1 bis 99 Gew.-%, bevorzugt 5 bis 90 Gew.-% und besonders bevorzugt 10 bis 80 Gew.-% des organischen Bindemittels a) enthält.

3.   Dentalmaterial nach Anspruch 1 oder 2, **dadurch gekennzeichnet, dass** es 0,1 bis 90 Gew.-%, bevorzugt 1 bis 80 Gew.-% und besonders bevorzugt 10 bis 60 Gew.-% des nanoskaligen Füllstoffs b) enthält.

4.   Dentalmaterial gemäß einem der Ansprüche 1 bis 3, **dadurch gekennzeichnet, dass** es 0,1 bis 95 Gew.-%, bevorzugt 1 bis 90 Gew.-% und besonders bevorzugt 10 bis 80 Gew.-% des anorganischen und/oder organischen Füllstoffs c) enthält.

**5.** Dentalmaterial gemäß einem der Ansprüche 1 bis 4, **dadurch gekennzeichnet, dass** es zusätzlich pyrogene und/oder nassgefällte Kieselsäuren zur Einstellung der Viskosität enthält.

**6.** Dentalmaterial gemäß Anspruch 5, **dadurch gekennzeichnet, dass** es 0 bis 30 Gew.-%, bevorzugt 0 bis 20 Gew.-% und besonders bevorzugt 0 bis 10 Gew.-% der pyrogene und/oder nassgefällte Kieselsäuren zur Einstellung der Viskosität enthält.

**7.** Dentalmaterial gemäß einem der Ansprüche 1 bis 6, **dadurch gekennzeichnet, dass** das organische Bindemittel a) eine Verbindung oder eine Mischung mehrerer Verbindungen ist, die radikalisch und/oder kationisch und/oder anionisch polymerisierbare Gruppen und/oder Gruppen, die eine Aushärtung über eine Kondensations-, Additions- und/oder Säure-Base-Reaktion erlauben, enthält.

**8.** Dentalmaterial gemäß einem der Ansprüche 1 bis 7, **dadurch gekennzeichnet, dass** der nanoskalige Füllstoff b) ein Metall-, Halbmetall- oder Mischmetalloxid, -Silikat, -Nitrid, -Sulfat, -Titanat, -Zirkonat, -Stannat, -Wolframat oder eine Mischung aus diesen Verbindungen ist.

**9.** Dentalmaterial gemäß Anspruch 8, **dadurch gekennzeichnet, dass** der nanoskalige Füllstoff b) Siliziumdioxid ist.

**10.** Dentalmaterial gemäß einem der Ansprüche 1 bis 9, **dadurch gekennzeichnet, dass** der Füllstoff c) ein sphärischer Füllstoff, Quarzpulver, Glaspulver, Glaskeramikpulver oder eine Mischung aus diesen Pulvern ist.

**11.** Dentalmaterial gemäß einem der Ansprüche 1 bis 9, **dadurch gekennzeichnet, dass** der anorganische und/oder organische Füllstoff c) ein gefülltes oder ungefülltes Splitterpolymerisat und/oder Perlpolymerisat ist.

**12.** Dentalmaterial gemäß einem der Ansprüche 1 bis 11, **dadurch gekennzeichnet, dass** der anorganische und/oder organische Füllstoff c) oberflächenmodifiziert ist und funktionelle Gruppen auf seiner Oberfläche besitzt, die mit dem organischen Bindemittel a) chemisch reagieren können oder eine hohe Affinität zu dem organischen Bindemittel a) haben.

**13.** Dentalmaterial gemäß einem der Ansprüche 1 bis 12, **dadurch gekennzeichnet, dass** es zusätzlich einen Initiator bzw. mehrere Initiatoren und optional einen Coinitiator bzw. mehrere Coinitiatoren enthält.

**14.** Dentalmaterial gemäß einem der Ansprüche 1 bis 13, **dadurch gekennzeichnet, dass** es röntgenopak ist.

**15.** Dentalmaterial nach einem der Ansprüche 1 bis 14, **dadurch gekennzeichnet, dass** es ein prothetisches, konservierendes und/oder präventives Dentalmaterial wie z.B. ein Zahnfüllungsmaterial, Stumpfaufbaumaterial, Material für provisorische Kronen und Brücken, Zahnzement, Adhäsiv, Material für künstliche Zähne, Verblendmaterial, Versiegelungsmaterial und Dentallack ist.

**16.** Verfahren zur Herstellung von Dentalmaterialien, mit den Schritten:

a) zur Verfügung stellen von:

a1) einem organischem Bindemittel,
a2) einem wenigstens teilweise agglomerierten und/oder aggregierten nanoskaligen Füllstoff,
a3) einem Mittel zur organischen Oberflächenmodifikation des Füllstoffs a2),
a4) wenigstens einem anorganischen und/oder organischen Füllstoff ausgewählt aus der Gruppe bestehend aus einem gemahlenen Füllstoff mit einer mittleren Korngröße zwischen 0,2 $\mu$m und 50 $\mu$m und einem sphärischen Füllstoff mit einer mittleren Korngröße zwischen 0,1 $\mu$m und 50 $\mu$m;

b) Durchführung einer organischen Oberflächenmodifikation des Füllstoffs a2) mit dem Mit-tel a3);
c) Einarbeiten des oberflächenmodifizierten nanoskaligen Füllstoffs in das organische Bindemittel bis wenigstens 50 Gew.-%, vorzugsweise wenigstens 60 Gew.-%, weiter vorzugsweise wenigstens 80 Gew.-% des nanoskaligen Füllstoffs einen Teilchendurchmesser von weniger als 200 nm, bevorzugt kleiner als 150 nm, besonders bevorzugt kleiner als 100 nm aufweisen;
d) Einarbeiten des Füllstoffs a4) in das organische Bindemittel;

wobei die Schritte c) und d) in beliebiger Reihenfolge oder gleichzeitig durchgeführt werden können und wobei Schritt b) vor oder gleichzeitig mit den Schritten c) und/oder d) durchgeführt wird.

17. Verfahren nach Anspruch 16, **dadurch gekennzeichnet, dass** die organische Oberflächenmodifizierung der nanoskaligen Füllstoffe a2) direkt im organischen Bindemittel durchgeführt wird.

18. Verfahren nach Anspruch 16 oder 17, **dadurch gekennzeichnet, dass** in Schritt b) zusätzliche mechanische Energie eingebracht wird, bevorzugt durch einen Hochgeschwindigkeitsrührer, einen Dissolver, eine Perlmühle oder einen Mischer.

19. Verfahren nach einem der Ansprüche 16 bis 18, **dadurch gekennzeichnet, dass** das organische Bindemittel a1) eine Verbindung oder eine Mischung mehrerer Verbindungen ist, die radikalisch und/oder kationisch und/oder anionisch polymerisierbare Gruppen und/oder Gruppen, die eine Aushärtung über eine Kondensations-, Additions und/oder Säure-Base-Reaktion erlauben, enthält.

20. Verfahren nach einem der Ansprüche 16 bis 19, **dadurch gekennzeichnet, dass** der nanoskalige Füllstoff a2) ein Metall-, Halbmetall- oder Mischmetalloxid, -Silikat, -Nitrid, -Sulfat, -Titanat, -Zirkonat, -Stannat, -Wolframat oder eine Mischung aus diesen Verbindungen ist.

21. Verfahren nach Anspruch 20, **dadurch gekennzeichnet, dass** der nanoskalige Füllstoff a2) Siliziumdioxid ist.

22. Verfahren nach einem der Ansprüche 16 bis 21, **dadurch gekennzeichnet, dass** bei der organischen Oberflächenmodifizierung Gruppen auf die Oberfläche der nanoskaligen Füllstoffe a2) eingeführt werden, die mit dem organischen Bindemittel a1) chemisch reagieren können oder eine hohe Affinität zu dem organischen Bindemittel haben.

23. Verfahren nach einem der Ansprüche 16 bis 22, **dadurch gekennzeichnet, dass** das zur organischen Oberflächenmodifizierung eingesetzte Mittel ein Silan, Chlorsilan, Silazan, Titanat, Zirkonat und/oder Wolframat ist.

24. Verfahren nach einem der Ansprüche 16 bis 23, **dadurch gekennzeichnet, dass** der anorganische und/oder organische Füllstoff a4) ein sphärischer Füllstoff, Quarzpulver, Glaspulver, Glaskeramikpulver oder eine Mischung aus diesen Pulvern ist.

25. Verfahren nach einem der Ansprüche 16 bis 23, **dadurch gekennzeichnet, dass** der anorganische und/oder organische Füllstoff a4) ein gefülltes oder ungefülltes Splitterpolymerisat und/oder Perlpolymerisat ist.

26. Verfahren nach einem der Ansprüche 16 bis 25, **dadurch gekennzeichnet, dass** der anorganische und/oder organische Füllstoff a4) organisch oberflächenmodifiziert ist und damit funktionelle Gruppen auf seiner Oberfläche besitzt, die mit dem organischen Bindemittel a1) chemisch reagieren können oder eine hohe Affinität zu dem organischen Bindemittel haben.

**Claims**

1. A filled and polymerisable dental material, **characterised in that** it contains:

   a) an organic binder,
   b) a nanoscale filler which exhibits the following features:

   - at least 50 wt.%, preferably at least 60 wt.% and particularly preferably at least 80 wt.% of the nanoparticles have a particle diameter of less than 200 nm, preferably of less than 150 nm, particularly preferably of less than 100 nm,
   - at least 20 particle number %, preferably at least 30 particle number %, preferably at least 40 particle number % and particularly preferably at least 50 particle number % of the nanoparticles are aggregated particles,
   - the nanoscale filler is organically surface-modified,

   c) at least one inorganic and/or organic filler selected from the group consisting of a ground filler with an average grain size of between 0.2 μm and 50 μm and a spherical filler with an average grain size of between 0.1 μm

and 50 μm.

2. A dental material according to claim 1, **characterised in that** it contains 1 to 99 wt.%, preferably 5 to 90 wt.% and particularly preferably 10 to 80 wt.% of the organic binder a).

3. A dental material according to claim 1 or claim 2, **characterised in that** it contains 0.1 to 90 wt.%, preferably 1 to 80 wt.% and particularly preferably 10 to 60 wt.% of the nanoscale filler b).

4. A dental material according to any one of claims 1 to 3, **characterised in that** it contains 0.1 to 95 wt.%, preferably 1 to 90 wt.% and particularly preferably 10 to 80 wt.% of the inorganic and/or organic filler c).

5. A dental material according to any one of claims 1 to 4, **characterised in that** it additionally contains pyrogenic and/or wet-precipitated silicas to adjust viscosity.

6. A dental material according to claim 5, **characterised in that** it contains 0 to 30 wt.%, preferably 0 to 20 wt.% and particularly preferably 0 to 10 wt.% of pyrogenic and/or wet-precipitated silicas to adjust viscosity.

7. A dental material according to any one of claims 1 to 6, **characterised in that** the organic binder a) is a compound or a mixture of two or more compounds which contains free-radically and/or cationically and/or anionically polymerisable groups and/or groups which permit curing by means of a condensation, addition and/or acid-base reaction.

8. A dental material according to any one of claims 1 to 7, **characterised in that** the nanoscale filler b) is a metal, semimetal or mischmetal oxide, silicate, nitride, sulfate, titanate, zirconate, stannate, tungstate or a mixture of these compounds.

9. A dental material according to claim 8, **characterised in that** the nanoscale filler b) is silicon dioxide.

10. A dental material according to any one of claims 1 to 9, **characterised in that** the filler c) is a spherical filler, quartz powder, glass powder, glass-ceramic powder or a mixture of these powders.

11. A dental material according to any one of claims 1 to 9, **characterised in that** the inorganic and/or organic filler c) is a filled or unfilled splinter polymer and/or bead polymer.

12. A dental material according to any one of claims 1 to 11, **characterised in that** the inorganic and/or organic filler c) is surface-modified and has functional groups on its surface which are capable of reacting chemically with the organic binder a) or have an elevated affinity for the organic binder a).

13. A dental material according to any one of claims 1 to 12, **characterised in that** it additionally contains an initiator or two or more initiators and optionally a coinitiator or two or more coinitiators.

14. A dental material according to any one of claims 1 to 13, **characterised in that** it is X-ray opaque.

15. A dental material according to any one of claims 1 to 14, **characterised in that** it is a prosthetic, conservative and/or preventive dental material such as for example a dental filling material, core build-up material, material for temporary crowns and bridges, dental cement, adhesive, material for artificial teeth, overlay material, sealing material and dental lacquer.

16. A method for producing dental materials comprising the steps of:

    a) providing

        a1) an organic binder,
        a2) an at least partially agglomerated and/or aggregated nanoscale filler,
        a3) an agent for organically surface-modifying the filler a2),
        a4) at least one inorganic and/or organic filler selected from the group consisting of a ground filler with an average grain size of between 0.2 μm and 50 μm and a spherical filler with an average grain size of between 0.1 μm and 50 μm;

b) carrying out organic surface modification of the filler a2) with the agent a3);

c) incorporating the surface-modified nanoscale filler into the organic binder until at least 50 wt.%, preferably at least 60 wt.%, further preferably at least 80 wt.% of the nanoscale filler have a particle diameter of less than 200 nm, preferably of less than 150 nm, particularly preferably of less than 100 nm;

d) incorporating the filler a4) into the organic binder;

wherein steps c) and d) may be carried out in any desired order or simultaneously and wherein step b) is carried out before or simultaneously with steps c) and/or d).

17. A method according to claim 16, **characterised in that** organic surface modification of the nanoscale fillers a2) is carried out directly in the organic binder.

18. A method according to claim 16 or claim 17, **characterised in that** additional mechanical energy is introduced in step b), preferably by a high-speed stirrer, a dissolver, a bead mill or a mixer.

19. A method according to any one of claims 16 to 18, **characterised in that** the organic binder a1) is a compound or a mixture of two or more compounds which contains free-radically and/or cationically and/or anionically polymerisable groups and/or groups which permit curing by means of a condensation, addition and/or acid-base reaction.

20. A method according to any one of claims 16 to 19, **characterised in that** the nanoscale filler a2) is a metal, semimetal or mischmetal oxide, silicate, nitride, sulfate, titanate, zirconate, stannate, tungstate or a mixture of these compounds.

21. A method according to claim 20, **characterised in that** the nanoscale filler a2) is silicon dioxide.

22. A method according to any one of claims 16 to 21, **characterised in that**, during organic surface modification, groups are introduced onto the surface of the nanoscale fillers a2) which are capable of reacting chemically with the organic binder a1) or have an elevated affinity for the organic binder.

23. A method according to any one of claims 16 to 22, **characterised in that** the agent used for organic surface modification is a silane, chlorosilane, silazane, titanate, zirconate and/or tungstate.

24. A method according to any one of claims 16 to 23, **characterised in that** the inorganic and/or organic filler a4) is a spherical filler, quartz powder, glass powder, glass-ceramic powder or a mixture of these powders.

25. A method according to any one of claims 16 to 23, **characterised in that** the inorganic and/or organic filler a4) is a filled or unfilled splinter polymer and/or bead polymer.

26. A method according to any one of claims 16 to 25, **characterised in that** the inorganic and/or organic filler a4) is organically surface-modified and so has functional groups on its surface which are capable of reacting chemically with the organic binder a1) or have an elevated affinity for the organic binder.

**Revendications**

1. Matériau dentaire chargé et polymérisable, **caractérisé en ce qu'**il contient :

a) un liant organique,
b) une charge nanométrique, qui présente les caractéristiques suivantes :

- au moins 50% en poids, de préférence au moins 60% en poids et de manière particulièrement préférée, au moins 80% en poids des nanoparticules présentent un diamètre des particules inférieur à 200 nm, de préférence inférieur à 150 nm, de manière particulièrement préférée inférieur à 100 nm,
- au moins 20% du nombre de particules, de préférence au moins 30% du nombre de particules, de préférence au moins 40% du nombre de particules et de manière particulièrement préférée, au moins 50% du nombre de particules des nanoparticules sont des particules agrégées,
- la charge nanométrique est modifiée en surface de manière organique,

c) au moins une charge inorganique et/ou organique, choisie parmi le groupe consistant en une charge broyée avec une taille moyenne des grains allant de 0,2 μm à 50 μm et une charge sphérique avec une taille moyenne des grains allant de 0,1 μm à 50 μm.

2. Matériau dentaire selon la revendication 1, **caractérisé en ce qu'**il contient 1 à 99% en poids, de préférence 5 à 90% en poids et de manière particulièrement préférée, 10 à 80% en poids du liant organique a).

3. Matériau dentaire selon la revendication 1 ou 2, **caractérisé en ce qu'**il contient 0,1 à 90% en poids, de préférence 1 à 80% en poids et de manière particulièrement préférée, 10 à 60% en poids de la charge nanométrique b).

4. Matériau dentaire selon l'une des revendications 1 à 3, **caractérisé en ce qu'**il contient 0,1 à 95% en poids, de préférence 1 à 90% en poids et de manière particulièrement préférée, 10 à 80% en poids de la charge inorganique et/ou organique c).

5. Matériau dentaire selon l'une des revendications 1 à 4, **caractérisé en ce qu'**il contient en outre, de l'acide silicique pyrogéné et/ou précipité par voie humide pour l'ajustement de la viscosité.

6. Matériau dentaire selon la revendication 5, **caractérisé en ce qu'**il contient 0 à 30% en poids, de préférence 0 à 20% en poids et de manière particulièrement préférée, 0 à 10% en poids de l'acide silicique pyrogéné et/ou précipité par voie humide pour l'ajustement de la viscosité.

7. Matériau dentaire selon l'une des revendications 1 à 6, **caractérisé en ce que** le liant organique a) est un composé ou un mélange de plusieurs composés, qui contient des groupes polymérisables de manière radicalaire et/ou cationique et/ou anionique et/ou des groupes qui permettent un durcissement par une réaction de condensation, d'addition et/ou acide-base.

8. Matériau dentaire selon l'une des revendications 1 à 7, **caractérisé en ce que** la charge nanométrique b) est un oxyde, silicate, nitrure, sulfate, titanate, zirconate, stannate, tungstate métallique, semi-métallique ou d'un métal des terres rares ou un mélange de ces composés.

9. Matériau dentaire selon la revendication 8, **caractérisé en ce que** la charge nanométrique b) est le dioxyde de silicium.

10. Matériau dentaire selon l'une des revendications 1 à 9, **caractérisé en ce que** la charge c) est une charge sphérique, la poudre de quartz, la poudre de verre, la poudre de céramique de verre ou un mélange de ces poudres.

11. Matériau dentaire selon l'une des revendications 1 à 9, **caractérisé en ce que** la charge inorganique et/ou organique c) est un polymère en éclats et/ou un polymère en perles chargés ou non chargés.

12. Matériau dentaire selon l'une des revendications 1 à 11, **caractérisé en ce que** la charge inorganique et/ou organique c) est modifiée en surface et possède des groupes fonctionnels sur sa surface, qui peuvent réagir chimiquement avec le liant organique a) ou qui ont une affinité élevée pour le liant organique a).

13. Matériau dentaire selon l'une des revendications 1 à 12, **caractérisé en ce qu'**il contient en outre, un initiateur et respectivement, plusieurs initiateurs et le cas échéant, un co-initiateur et respectivement, plusieurs co-initiateurs.

14. Matériau dentaire selon l'une des revendications 1 à 13, **caractérisé en ce qu'**il est opaque aux rayons X.

15. Matériau dentaire selon l'une des revendications 1 à 14, **caractérisé en ce qu'**il est un matériau dentaire prothétique à conserver et/ou préventif, comme par exemple un matériau de plombage, un matériau de réparation sur un moignon, un matériau pour couronnes et bridges provisoires, un ciment dentaire, un adhésif, un matériau pour dents artificielles, un matériau de revêtement, un matériau de vitrification et une laque dentaire.

16. Procédé de préparation de matériaux dentaires, comprenant les étapes consistant à :

    a) disposer de :

        a1) un liant organique,
        a2) une charge nanométrique au moins partiellement agglomérée et/ou agrégée,

a3) un agent pour la modification organique de la surface de la charge a2),

a4) au moins une charge inorganique et/ou organique choisie parmi le groupe consistant en une charge broyée ayant une taille moyenne des grains allant de 0,2 μm à 50 μm et une charge sphérique ayant une taille moyenne des grains allant de 0,1 μm à 50 μm ;

b) réaliser une modification organique de la surface de la charge a2) avec l'agent a3) ;

c) incorporer la charge nanométrique modifiée en surface au liant organique, où au moins 50% en poids, de préférence au moins 60% en poids, de manière encore préférée au moins 80% en poids de la charge nanométrique présentent un diamètre des particules inférieur à 200 nm, de préférence inférieur à 150 nm, de manière particulièrement préférée inférieur à 100 nm ;

d) incorporer la charge a4) dans le liant organique ;

où les étapes c) et d) peuvent être réalisées en ordre quelconque ou simultanément et où l'étape b) est réalisée avant ou simultanément aux étapes c) et/ou d).

17. Procédé selon la revendication 16, **caractérisé en ce que** la modification organique de la surface de la charge nanométrique a2) est réalisée directement dans le liant organique.

18. Procédé selon la revendication 16 ou 17, **caractérisé en ce que** l'énergie mécanique supplémentaire est introduite dans l'étape b), de préférence par un agitateur à haute vitesse, un disperseur, un moulin à bille ou un mélangeur.

19. Procédé selon l'une des revendications 16 à 18, **caractérisé en ce que** la liant organique a1) est un composé ou un mélange de plusieurs composés, qui contient des groupes polymérisables de manière radicalaire et/ou cationique et/ou anionique et/ou des groupes qui permettent un durcissement par une réaction de condensation, d'addition et/ou acide-base.

20. Procédé selon l'une des revendications 16 à 19, **caractérisé en ce que** la charge nanométrique a2) est un oxyde, silicate, nitrure, sulfate, titanate, zirconate, stannate, tungstate métallique, semi-métallique ou d'un métal des terres rares ou un mélange de ces composés.

21. Procédé selon la revendication 20, **caractérisé en ce que** la charge nanométrique a2) est le dioxyde de silicium.

22. Procédé selon l'une des revendications 16 à 21, **caractérisé en ce que** lors de la modification organique de surface, des groupes sont introduits sur la surface de la charge nanométrique a2), lesquels peuvent réagir chimiquement avec le liant organique a1) ou ont une affinité élevée pour la liant organique a1).

23. Procédé selon l'une des revendications 16 à 22, **caractérisé en ce que** l'agent mis en oeuvre pour la modification organique de surface est un silane, un chlorosilane, un silazane, un titanate, un zirconate et/ou un tungstate.

24. Procédé selon l'une des revendications 16 à 23, **caractérisé en ce que** la charge inorganique et/ou organique a4) est une charge sphérique, la poudre de quartz, la poudre de verre, la poudre de céramique de verre ou un mélange de ces poudres.

25. Procédé selon l'une des revendications 16 à 23, **caractérisé en ce que** la charge inorganique et/ou organique a4) est un polymère en éclats et/ou un polymère en perles chargés ou non chargés.

26. Procédé selon l'une des revendications 16 à 25, **caractérisé en ce que** la charge inorganique et/ou organique a4) est modifiée de manière organique en surface et possède ainsi, des groupes fonctionnels sur sa surface, qui peuvent réagir chimiquement avec le liant organique a1) ou qui ont une affinité élevée pour le liant organique.

**Figur 1**

**Figur 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 02092022 A
- DE PS3247800 C
- US 6387981 B

**In der Beschreibung aufgeführte Nicht-Patentliteratur**

- **D.C. SMITH.** *Biomaterials,* 1998, vol. 19, 467-478
- **A.J. DEGEE et al.** *J. Dent. Res.,* 1986, vol. 65 (5), 654-658